# EUROPEAN PATENT SPECIFICATION

(11) **EP 1 654 241 B1**
(45) Date of publication and mention of the grant of the patent: **19.03.2008**
(21) Application number: 04740596.4
(22) Date of filing: 02.07.2004
(51) Int. Cl.: C07D 273/02, C07D 291/08, A61P 25/00, A61K 31/33

(54) **MACROCYCLIC COMPOUNDS HAVING ASPARTIC PROTEASE INHIBITING ACTIVITY AND PHARMACEUTICAL USES THEREOF**
MAKROZYKLISCHE VERBINDUNGEN MIT ASPARAGINPROTEASEHEMMENDER WIRKUNG UND DEREN PHARMAZEUTISCHE VERWENDUNG
COMPOSES MACROCYLIQUES PRESENTANT UNE ACTIVITE D'INHIBITION DE PROTEASE ASPARTIQUE ET UTILISATIONS PHARMACEUTIQUES DE CEUX-CI

(30) Priority: 03.07.2003 GB 0315654
(43) Date of publication of application: 10.05.2006
(73) Proprietor: Novartis AG, 4056 Basel (CH); Novartis Pharma GmbH, 1230 Vienna (AT)
(72) Inventor: BETSCHART, Claudia, CH-4054 Basel (CH); TINTELNOT-BLOMLEY, Marina, 79689 Maulburg (DE)
(74) Representative: Leon, Susanna Iris
(86) International application number: PCT/EP2004/007247
(87) International publication number: WO 2005/003106

(56) References cited:
- US-B1- 6 184 241
- SMITH R A ET AL: "DESIGN, SYNTHESIS, AND ACTIVITY OF CONFORMATIONALLY-CONSTRAINED MACROCYCLIC PEPTIDE-BASED INHIBITORS OF HUV PROTEASE" BIOORGANIC & MEDICINAL CHEMISTRY LETTERS, OXFORD, GB, vol. 4, no. 18, 1994, pages 2217-2222, XP000654258 ISSN: 0960-894X
- MEYER; BARTLETT: "Macrocyclic Inhibitors of Penicillopepsin" J. AM. CHEM. SOC., vol. 120, no. 19, 1998, pages 4600-4609, XP002301583

## Description

The present invention relates to novel macrocyclic compounds, their preparation, their use as pharmaceuticals and pharmaceutical compositions containing them.

More particularly the invention provides compounds of formula I 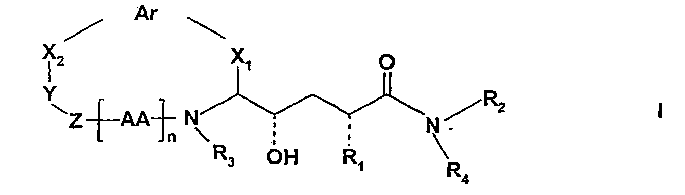 wherein
- R₁: is (C₁₋₈)alkyl, (C₁₋₄)alkoxy(C₁₋₄)alkyl, hydroxy(C₁₋₆)alkyl, (C₁₋₄)alkylthio(C₁₋₄)alkyl, (C₁₋₆)alkenyl, (C₃₋₇)cycloalkyl, (C₃₋₇)cycloalkyl(C₁₋₄)alkyl, piperidinyl or pyrrolidinyl,
- R₂ and R₄,: independently, are hydrogen or optionally substituted (C₁₋₈)alkyl, (C₃₋₇) cycloalkyl, (C₃₋₇)cycloalkyl(C₁₋₄)alkyl, aryl, aryl(C₁₋₄)alkyl, heteroaryl or heteroaryl(C₁₋₄) alkyl, or
- R₂ and R₄,: together with the nitrogen to which they are attached, form an optionally substituted piperidino, pyrrolidinyl, morpholino or piperazinyl group,
- R₃: is hydrogen or (C₁₋₄)alkyl,
- X₁: is CH₂,
- X₂: is CH₂, O, S, CO, COO, OCO, NHCO, CONH, or NR, R being hydrogen or (C₁₋₄)alkyl,
- Y: is (C₁₋₈)alkylen or (C₁₋₈)alkylenoxy(C₁₋₆)alkylen, (C₁₋₈)alkenylen or (C₁₋₈)alkenylenoxy (C₁₋₆)alkylen,
- Ar: is a phenyl ring optionally mono- di- or trisubstituted by, independently, hydroxy or halogen, whereby X₁ and X₂ are in meta or para position to each other,
and either
- Z: is CO,
- AA: is a natural or unnatural alpha-amino-acid, and
- n: is 0 or 1,
or
- Z: is SO₂,
- AA: is an optionally substituted ethylencarbonyl group (derived from a natural or unnatural alpha-amino acid by replacement of the nitrogen by a methylen group), and
- n: is 1
in free base or acid addition salt form.

Halogen denotes fluorine, bromine, chlorine or iodine.

When R₂ and/or R₄ is substituted alkyl or cycloalkyl, or together with the nitrogen to which they are attached, form a substituted piperidino, pyrrolidinyl, morpholino or piperazinyl group, substituents may be one to three groups selected from hydroxy, hydroxy(C₁₋₄)alkyl, (C₁₋₄)alkoxy, (C₁₋₄)alkoxy(C₁₋₄)alkyl, (C₁₋₄)alkoxy(C₁₋₄)alkoxy, (C₁₋₄)alkylsulfanyl, (C₁₋₄)alkoxycarbonyl, (C₁₋₄)alkylcarbonyloxy, (C₁₋₄)alkylcarbonylamino, (C₁₋₄)alkylcarbonyl, (C₁₋₄)sulfonyl, cyano, oxo, hetero (C₃₋₇)cycloalkyl or heteroaryl.

When R₂ and/or R₄ is substituted aryl or heteroaryl, substituents may be one to three groups selected from halogen, hydroxy, cyano, trifluoromethyl, carboxy, (C₁₋₄)alkyloxycarbonyl, (C₁₋₄)alkylcarbamoyl, (C₁₋₄)alkylsulfonyl, (C₁₋₄)alkylcarbonyloxy, (C₁₋₄)alkylcarbonyl, (C₁₋₄)alkyl, (C₁₋₄)alkoxy or hydroxy(C₁₋₄)alkyl.

Aryl is an aromatic 6-membered ring optionally mono-, di- or tri-substitueted by, independently, hydroxy, cyano, trifluoromethyl, carboxy, (C₁₋₄)alkyloxycarbonyl, (C₁₋₄)alkylcarbamoyl, (C₁₋₄)alkylsulfonyl, (C₁₋₄)alkylcarbonyloxy, (C₁₋₄)alkylcarbonylamino, (C₁₋₄)alkylcarbonyl, (C₁₋₄)alkyl, (C₁₋₄)alkoxy or hydroxy(C₁₋₄)alkyl. It can also be fused with an cycloalkyl or additional aromatic or heteroaromatic ring (e.g. to form a naphthyl, quinolinyl, indolyl group).

Heteroaryl is an aromatic 5- or 6- membered ring in which 1, 2 or 3 atoms are heteroatoms independently selected from O, N and S, optionally mono- di- or tri- substituted by, independently, hydroxy or halogen. Heteroaryl is for example 1-methyl-1H-pyrrol-2-yl or 1 H-imidazol-2-yl. It can also be fused with an cycloalkyl or additional aromatic or heteroaromatic ring (e.g. to form a quinolinyl, indolyl group).

Any alkyl or alkoxy group is straight or branched and is preferably methyl or methoxy.

In formula I the following significances are preferred independently, collectively or in any combination or sub-combination:
R₁ is preferably (C₁₋₈)alkyl, more preferably (C₁₋₄)alkyl, in particular methyl, ethyl or propyl.
R₂ is preferably (C₁₋₈)alkyl, more preferably (C₁₋₆)alkyl, in particular methyl, ethyl or propyl, or butyl, such as n-butyl, sec-butyl or tert-butyl.
R₃ is preferably hydrogen.
R₄ is preferably hydrogen.
X₂ is preferably methylen or oxygen.
Y is preferably (C₁₋₈)alkylen, more preferably (C₃₋₆)alkylen.
Ar is preferably unsubstituted phenylen whereby X₁ and X₂ are in meta position to each other.
AA is preferably selected from -N(H)-CH(CH₃)-C(O)-, -CH₂-CH(CH₃)-C(O)- and -CH₂-CH₂-C(O)-.

A preferred embodiment of the present invention relates to compounds of formula I, wherein
- R₁: is (C₁₋₈)alkyl, (C₁₋₄)alkoxy(C₁₋₄)alkyl, hydroxy(C₁₋₆)alkyl, (C₁₋₄)alkylthio(C₁₋₄)alkyl, (C₁₋₆)alkenyl, (C₃₋₇)cycloalkyl, (C₃₋₇)cycloalkyl(C₁₋₄)alkyl, piperidinyl or pyrrolidinyl,
- R₂: and R₄, independently, are
(a) hydrogen
(b) (C₁₋₈)alkyl, (C₃₋₇) cycloalkyl or (C₃₋₇)cycloalkyl(C₁₋₄)alkyl, in each case optionally substituted by one to three groups selected from hydroxy, hydroxy(C₁₋₄)alkyl, (C₁₋₄)alkoxy, (C₁₋₄)alkoxy(C₁₋₄)alkyl, (C₁₋₄)alkoxy(C₁₋₄)alkoxy, (C₁₋₄)alkylsulfanyl, (C₁₋₄)alkoxycarbonyl, (C₁₋₄)alkylcarbonyloxy, (C₁₋₄)alkylcarbonylamino, (C₁₋₄)alkylcarbonyl, (C₁₋₄)sulfonyl, cyano, oxo, hetero (C₃₋₇)cycloalkyl or heteroaryl, or
(c) aryl, aryl(C₁₋₄)alkyl, heteroaryl or heteroaryl(C₁₋₄) alkyl, wherein in the latter two radicals heteroaryl denotes an aromatic 5- or 6- membered ring in which 1, 2 or 3 atoms are heteroatoms independently selected from O, N and S, wherein all radicals are optionally substituted by one to three groups selected from halogen, hydroxy, cyano, trifluoromethyl, carboxy, (C₁₋₄)alkyloxycarbonyl, (C₁₋₄)alkylcarbamoyl, (C₁₋₄)alkylsulfonyl, (C₁₋₄)alkylcarbonyloxy, (C₁₋₄)alkylcarbonyl, (C₁₋₄)alkyl, (C₁₋₄)alkoxy or hydroxy(C₁₋₄)alkyl, or
- R₂ and R₄,: together with the nitrogen to which they are attached, form an piperidino, pyrrolidinyl, morpholino or piperazinyl group, each of which is optionally substituted by one to three groups selected from hydroxy, hydroxy(C₁₋₄)alkyl, (C₁₋₄)alkoxy, (C₁₋₄)alkoxy(C₁₋₄)alkyl, (C₁₋₄)alkoxy(C₁₋₄)alkoxy, (C₁₋₄)alkylsulfanyl, (C₁₋₄)alkoxycarbonyl, (C₁₋₄)alkylcarbonyloxy, (C₁₋₄)alkylcarbonylamino, (C₁₋₄)alkylcarbonyl, (C₁₋₄)sulfonyl, cyano, oxo, hetero (C₃₋₇)cycloalkyl or heteroaryl,
- R₃: is hydrogen or (C₁₋₄)alkyl,
- X₁: is CH₂,
- X₂: is CH₂, O, S, CO, COO, OCO, NHCO, CONH, or NR, R being hydrogen or (C₁₋₄)alkyl,
- Y: is (C₁₋₈)alkylen or (C₁₋₈)alkylenoxy(C₁₋₆)alkylen, (C₁₋₈)alkenylen or (C₁₋₈)alkenylenoxy(C₁₋₆)alkylen,
- Ar: is a phenyl ring optionally mono- di- or trisubstituted by, independently, hydroxy or halogen, whereby X₁ and X₂ are in meta or para position to each other,
and either
- Z: is CO,
- AA: is a natural or unnatural alpha-amino-acid, and
- n: is 0 or 1,
or
- Z: is SO₂,
- AA: is an optionally substituted ethylencarbonyl group (derived from a natural or unnatural alpha-amino acid by replacement of the nitrogen by a methylen group), and
- n: is 1.

Especially preferred are compounds of formula I, wherein
- R₁: is (C₁₋₄)alkyl,
- R₂: is (C₁₋₆)alkyl,
- R₃: is hydrogen or (C₁₋₄)alkyl,
- R₄: is hydrogen,
- X₁: is CH₂,
- X₂: is CH₂ or O,
- Y: is (C₁₋₈)alkylen,
- Ar: is unsubstituted phenylen, whereby X₁ and X₂ are in meta position to each other,
and either
- Z: is CO,
- AA: is a natural or unnatural alpha-amino-acid, and
- n: is 0 or 1,
or
- Z: is SO₂,
- AA: is an optionally substituted ethylencarbonyl group (derived from a natural or unnatural alpha-amino acid by replacement of the nitrogen by a methylen group), and
- n: is 1.

More preferred are compounds of formula I, wherein
- R₁: is (C₁₋₄)alkyl,
- R₂: is (C₁₋₆)alkyl,
- R₃: is hydrogen or (C₁₋₄)alkyl,
- R₄: is hydrogen,
- X₁: is CH₂,
- X₂: is CH₂ or O,
- Y: is (C₃₋₆)alkylen,
- Ar: is unsubstituted phenylen whereby X₁ and X₂ are in meta position to each other,
and either
- Z: is CO,
- AA: is -N(H)-CH(CH₃)ₘ-C(O)-, wherein m is 0 or 1 and
- n: is 0 or 1,
or
- Z: is SO₂,
- AA: is -CH₂-CH(CH₃)-C(O)- or -CH₂-CH₂-C(O)- and
- n: is 1.

Most preferred are those compounds as described in the Examples.

In a further aspect, the invention provides a process for the production of the compounds of formula I and their salts, comprising the steps of
a) for the production of a compound of formula I wherein Z is CO, cyclisation by amide formation of a compound of formula II 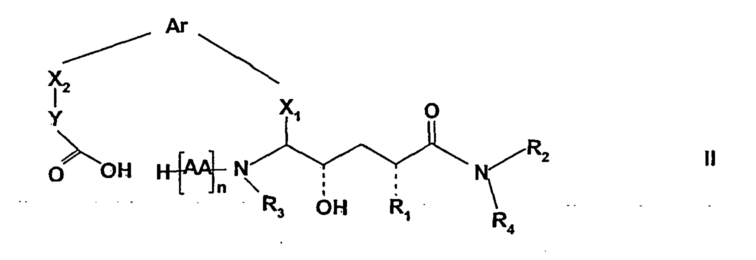 wherein R₁, R₂, R₃, R₄, X₁, X₂, Y, Ar, AA and n are as defined above,
b) for the production of a compound of formula I wherein Z is SO₂ and Y is (C₁₋₈)alkenylen or (C₁₋₈)alkenylenoxy(C₁₋₆)alkylen, cyclisation by metathesis of a compound of formula III 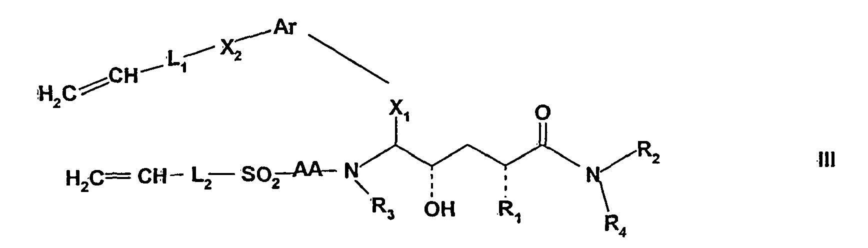 wherein R₁, R₂, R₃, R₄, X₁, X₂, Ar and AA are as defined above and L₁ and L₂, independently are alkylen or alkylenoxyalkylen groups, or
c) for the production of a compound of formula I wherein Z is SO₂ and Y is (C₁₋₈) alkylen or (C₁₋₈)alkylenoxy(C₁₋₆)alkylen, hydrogenation of a compound of formula I wherein Z is SO₂ and Y is
(C₁₋₈)alkenylen or (C₁₋₈)alkenylenoxy(C₁₋₆)alkylen,
and recovering the so obtained compound of formula I in free base or acid addition salt form.

The reactions can be effected according to known methods, for example the cyclisation of process A can be effected as described in Example 1.

Working-up the reaction mixtures and purification of the compounds thus obtained may be carried out in accordance to known procedures.

Acid addition salts may be produced from the free bases in known manner, and vice-versa.

The starting material of formula II may be produced for example as described in Example 1.

Compounds of formula I and their pharmaceutically acceptable acid addition salts, hereinafter referred to as agents of the invention, exhibit valuable pharmacological properties when tested in vitro and in animals, and are therefore useful as pharmaceuticals.

The agents of the invention are inhibitors of aspartic proteases and can be used for the treatment of disorders involving processing by such enzymes. Particularly they inhibit beta-secretase and as such inhibit the generation of beta-amyloid and the subsequent aggregation into oligomers and fibrils.

### Test 1 Inhibition of BACE

Recombinant BACE (extracellular domain, expressed in baculovirus and purified using standard methods) at 6 nM concentration is incubated with test compound at various concentrations for 1 hour at room temperature in 100 mM acetate buffer, pH 4.5, containing 0.1 % CHAPS. Synthetic peptide substrate Mca-Ser-Glu-Val-Asn-Leu-Asp-Ala-Glu-Phe-Lys(DNP) is added to a final concentration of 3 µM and increase in fluorescence is recorded at excitation of 325 nm and emission at 400 nm in a microplate spectro-fluorimeter for 20 minutes in 1-minute intervals. IC₅₀ values are calculated from percentage of inhibition of BACE-activity as a function of test compound concentration.

### Test 2 Inhibition of BACE-2

Recombinant BACE-2 (extracellular domain, expressed in baculovirus and purified using standard methods) at 2.5 nM concentration is incubated with test compound at various concentrations for 1 hour at room temperature in 100 mM acetate buffer, pH 4.5, containing 0.1 % CHAPS. Synthetic peptide substrate Mca-Ser-Glu-Val-Asn-Leu-Asp-Ala-Glu-Phe-Lys(DNP) is added to a final concentration of 3 µM and increase in fluorescence is recorded at excitation of 325 nm and emission at 400 nm in a microplate spectro-fluorimeter for 20 minutes in 1-minute intervals. IC₅₀ values are calculated from percentage of inhibition of BACE-2-activity as a function of test compound concentration.

### Test 3 Inhibition of Cathepsin D

Recombinant cathepsin D (expressed as procathepsin D in baculovirus, purified using standard methods and activated by incubation in sodium formate buffer pH 3.7) is incubated with test compound at various concentrations for 1 hour at room temperature in 100 mM sodium formate buffer, pH 3.1. Synthetic peptide substrate Mca-Gly-Lys-Pro-lle-Leu-Phe-Phe-Arg-Leu-Lys(DNP)-D-Arg-NH₂ is added to a final concentration of 2 µM and increase in fluorescence is recorded at excitation of 325 nm and emission at 400 nm in a microplate spectro-fluorimeter for 20 minutes in 1-minute intervals. lC₅₀ values are calculated from percentage of inhibition of cathepsin D-activity as a function of test compound concentration. In the described assay, the agents of the present invention show IC₅₀ values between about 0.2 and 5 µM, in particular between 0.2 and 2 µM.

### Test 4 Inhibition of cellular release of amyloid peptide 1-40

Chinese hamster ovary cells are transfected with the gene for amyloid precursor protein. Cells are plated at a density of 8000 cells/well in a 96- well microtiter plate and cultivated for 24 hours in DMEM cell culture medium containing 10 % FCS. Test compound is added to the cells at various concentrations, and cells are cultivated for 24 hours in presence of test compound. Supernatants are collected, and concentration of amyloid peptide 1-40 is determined using sandwich ELISA. Potency of the compound is calculated from the percentage of inhibition of amyloid peptide release as a function of test compound concentration.

### Test 5 Cytotoxicity

Cytotoxicity of test compound is determined using Chinese hamster ovary cells transfected with the gene for amyloid precursor protein. Cells are plated at a density of 8000 cells/well in a 96- well microtiter plate and cultivated for 24 hours in DMEM cell culture medium containing 10 % FCS. Test compound is added to the cells at various concentrations, and cells are cultivated for 24 hours in presence of test compound. After removing the supernatant, the percentage of living and dead cells is determined using a colorimetric readout (MTS-method), which measures mitochondrial dehydrogenase enzymes in living cells.

The agents of the invention are therefore useful e.g. for the treatment and/or prevention of neurological and vascular disorders related to beta-amyloid generation and/or aggregation such as neurodegenerative diseases like Alzheimer's disease, Down's Syndrome, memory and cognitive impairment, dementia, amyloid neuropathies, brain inflammation, nerve and brain trauma, vascular amyloidosis, or cerebral haemorrhage with amyloidosis.

Some of the agents of the invention also inhibit BACE2 (beta-site APP-cleaving enzyme 2) or Cathepsin D, close homologues of beta-secretase. Due to the correlation of BACE2 and CathD expression with a more tumorigenic and metastatic potential of tumor cells, such inhibitors are useful for the suppression of the metastasis process associated with tumor cells.

For the above-mentioned indications, the appropriate dosage will of course vary depending upon, for example, the compound employed, the host, the mode of administration and the nature and severity of the condition being treated. However, in general, satisfactory results in animals are indicated to be obtained at a daily dosage of from about 0.1 to about 100, preferably from about 1 to about 50 mg/kg animal body weight. In larger mammals, for example humans, an indicated daily dosage is in the range from about 10 to about 2000, preferably from about 10 to about 200 mg of an agent of the invention conveniently administered, for example, in divided doses up to four times a day or in sustained release form.

The agent of the invention may be administered by any conventional route, in particular enterally, preferably orally, for example in the form of tablets or capsules, or parenterally, for example in the form of injectable solutions or suspensions.

In accordance with the foregoing, the present invention also provides an agent of the invention, for use as a pharmaceutical, e.g. for the treatment of neurological and vascular disorders related to beta-amyloid generation and/or aggregation.

The present invention furthermore provides a pharmaceutical composition comprising an agent of the invention in association with at least one pharmaceutical carrier or diluent. Such compositions may be manufactured in conventional manner. Unit dosage forms contain, for example, from about 1 to about 1000, preferably from about 1 to about 500 mg of an agent of the invention.

The agents of the invention can be administered alone or in combination with other pharmaceutical agents effective, in the treatment of conditions mentioned above. The other pharmaceutical agents can be selected especially from donepezil hydrochloride, e.g., in the form as marketed under the trademark Aricept^{™}, rivastigmine, e.g., in the form as marketed, e.g. under the trademark Exelon^{™} and galantamine hydrobromide e.g., in the form as marketed, e.g. under the trademark Reminyl^{™}.

The pharmaceutical combination may be in form of a unit dosage form, whereby each unit dosage will comprise a predetermined amount of the two components, in admixture with suitable pharmaceutical carriers or diluents. Alternatively, the combination may be in form of a package containing the two components separately, e.g. a pack or dispenser-device adapted for the concomitant or separate administration of the two active agents, wherein these agents are separately arranged.

Moreover the present invention provides the use of an agent of the invention, for the manufacture of a medicament for the treatment of any neurological and vascular disorders related to beta-amyloid generation and/or aggregation.

In still a further aspect the present invention provides a method for the treatment of any neurological and vascular disorders related to beta-amyloid generation and/or aggregation, in a subject in need of such treatment, which comprises administering to such subject a therapeutically effective amount of an agent of the invention.

The following examples illustrate the invention.

### General abbreviations:

- BOC: tert-butoxycarbonyl
- BOP: benotriazol-1-yloxytris(dimethylamino)-phosphonium hexafluorophosphate
- DMPU: N, N'-dimethylpropyleneurea
- EDCI: 1-ethyl-3-[3-(dimethylamino)propyl]-carbodiimide hydrochloride
- EtOAc: ethylacetate
- h: hours
- HOBt: hydroxybenztriazole
- HPLC: high pressure liquide chromatography
- min: minutes
- MS: mass spectroscopy
- rt: room temperature
- TFA: trifluoroacetic acid
- THF: tetrahydrofuran

### Example 1: (2R,4S)-N-Butyl-4-hydroxy-2-methyl-4-((10S,13S)-10-methyl-8,11-dioxo-2-oxa-9,12-diaza-bicyclo[13.3.1]nonadeca-1(18),15(19),16-trien-13-yl)-butyramide

### a) [1-Benzenesulfonyl-2-(3-benzyloxy-phenyl)-ethyl]-carbamic acid tert-butyl ester

A suspension of (3-Benzyloxy-phenyl)-acetaldehyde (20.6g, 91mmol), tert-butylcarbamate (10.7g, 91 mmol, 1eq), sodium benzenesulfinate (18.3g, 109mmol, 1.2eq) and formic acid (5.2ml, 137mmol, 1.5eq) in 155 ml acetonitrile is stirred at 80°C for 4 h. After cooling to rt the mixture is taken up in EtOAc. The solution is washed with bicarbonate and brine, dried over magnesium sulfate and evaporated. The residue (37.3g) is used for the next step without further purification.

### b) [(S*)-2-(3-Benzyloxy-phenyl)-1-((S*)-5-oxo-2,5-dihydro-furan-2-yl)-ethyl]-carbamic acid tert-butyl ester

5H-Furan-2-one (11.2ml, 160mmol, 2eq) in THF (60ml) is added slowly to a solution of lithium diisopropylamide (80ml commercial 2M solution in THF/heptane/ethylbenzene, 160mmol, 2eq) in THF (180ml) at -78°C. The mixture is stirred for another 20min at -78°C before [1-Benzenesulfonyl-2-(4-benzyloxy-phenyl)-ethyl]-carbamic acid tert-butyl ester (37.3g, 80mmol) in THF (220ml) is added at the same temperature. After stirring for another 45 min at -78°C aqueous bicarbonate solution is added and the reaction mixture is taken up into EtOAc. The organic layer is washed with bicarbonate and brine and dried over magnesium sulfate. Evaporation of the solvent gives a residue that is purified by chromatography on silica using hexan/EtOAc 9/1 to 7/3. The product is recrystallized from ether/hexane to give the product as white crystals.
1 H-NMR (400MHz, CDCl3): 7.45-7.2 (m, 7H), 6.9-6.85 (m, 3H), 6.06 (d, 1 H), 5.07 (s, 2H), 4.90 (d, 1 H), 4.50 (d, 1 H), 4.20 (q, 1 H), 3.01 (dd, 1 H), 2.91 (dd, 1 H), 1.38 (s, 9H).

### c) [(S*)-2-(3-Benzyloxy-phenyl)-1-((S*)-5-oxo-tetrahydro-furan-2-yl)-ethyl]-carbamic acid tert-butyl ester

[(S*)-2-(4-Benzyloxy-phenyl)-1-((S*)-5-oxo-2,5-dihydro-furan-2-yl)-ethyl]-carbamic acid tert-butyl ester (11.1g, 27mmol) is hydrogenated (1atm H₂) at rt in THF (550ml) with Pt/C as catalyst (5% Engelhard 4709, 2.3g) during 1h. The catalyst is filtered off and the filtrate is evaporated. Purification by chromatography on silica (Flashmaster, hexane to hexane/EtOAc 55/45 over 40min) gives the product as yellowish oil.
1H-NMR (400MHz, CDCl3): 7.45-7.2 (m, 6H), 6.9-6.8 (m, 3H), 5:06 (s, 2H), 4.61 (d, 1H), 4.44 (t, 1 H), 4.00 (q, 1 H), 2.95 (dd, 1H), 2.85 (dd, 1H), 2.6-2.45 (m, 2H), 2.15-2.1 (m, 2H), 1.42 (s, 9H).

### d) [(S*)-2-(3-Benzyloxy-phenyl)-1-((2S*,4R*)-4-methyl-5-oxo-tetrahydro-furan-2-yl)-ethyl]-carbamic acid tert-butyl ester

To a solution of [(S*)-2-(4-Benzyloxy-phenyl)-1-((S*)-5-oxo-2,5-dihydro-furan-2-yl)-ethyl]-carbamic acid tert-butyl ester (11.4g, 27.7mmol) in THF (35ml) and DMPU (5ml, 42mmol, 1.5eq) at -78°C is added dropwise lithium-bis-(trimethylsilyl)-amide (55ml 1M solution in THF, 55mmol, 2eq). After stirring at -78°C for another 45 min methyliodide is added dropwise and the mixture is stirred another 3h at -78°C. Propionic acid (10.3 ml, 138mmol, 5eq) is added followed by water (10ml). After warming up to 0°C a 10% solution of citric acid (72ml) is added. The reaction mixture is extracted with EtOAc. The organic layer is washed with bicarbonate, 0.1N sodium sulfite and brine, dried over magnesium sulfate and evaporated. Purification by chromatography on silica (hexane/EtOAc 9/1 to 4/1) followed by recrystallization from ether/hexane gives white crystals.
MS (LC/MS): 448 (M+Na)
1 H-NMR (400MHz, CDCl3): 7.45-7.2 (m, 6H), 6.9-6.8 (m, 3H), 5.05 (s, 2H), 4.53 (d, 1H), 4.45 (t, 1H), 4.00 (q, 1 H), 2.93-2.85 (m, 2H), 2.74-2.68 (m, 1H), 2.41-2.34 (m, 1 H), 1.89-1.82 (m, 1H), 1.41 (s, 9H), 1.26 (d, 3H).

### e) [(1S*,2S*,4R*)-1-(3-Benryloxy-benzyt)-4-butylcarbamoyl-2-hydroxy-pentyl]-carbamic acid tert-butyl ester

[(S*)-2-(3-Benzyloxy-phenyl)-1-((25*,4R*)-4-methyl-5-oxo-tetrahydro-furan-2-yl)-ethyl]-carbamic acid tert-butyl ester (4.0g, 9.4mmol) is dissolved in butylamine (200ml) and stirred for 18 h in a heating bath of 90°C. The butylamine is evaporated and the residue is recrystallized from dichloromethane/ether/hexane to give white crystals.
MS (LC/MS): 521 (M+Na)
1 H-NMR (400MHz, CDCl3): 7.45-7.15 (m, 6H), 6.9-6.8 (m, 3H), 5.91 (s, 1 H), 5.04 (s, 2H), 4.89 (d, 1 H), 3.7-3.6 (m, 2H), 3.3-3.1 (m, 2H), 2.9-2.85 (m, 2H), 2.6-2.5 (m, 1 H), 1.75-1.6 (m, 2H), 1.5-1.25 (m, 4H), 1.41 (s, 9H), 1.12 (d, 3H), 0.92 (t, 3H).

### f) {(S)-1-[(1S*,25*,4R*)-1-(3-Benzyloxy-benzyl)-4-butylcarbamoyl-2-hydroxypentylcarbamoyl]-ethyl}-carbamic acid tert-butyl ester

[(1S*,2S*,4R*)-1-(3-Benzyloxy-benzyl)-4-butylcarbamoyl-2-hydroxy-pentyl]-carbamic acid tert-butyl ester (4.4g, 8.8mmol) is dissolved in trifluoroacetic acid (95ml) and stirred at rt during 1.25h. The solvent is evaporated and the residue is a few times redissolved in ether and dried again to give a white foam (3.92g, 9.0mmol). The foam is dissolved in dichloromethane (210ml) and BOC-L-alanine (1.05g, 10.8mmol, 1.2eq), EDCl (2.59g, 13.5mmol, 1.5eq), HOBt (1.46g, 10.8mmol, 1.2eq) and triethylamine (3.76g, 27mmol, 3eq) are added. The reaction is stirred at rt (with an argon balloon) for 18 h. The mixture is extracted with EtOAc and the organic layer is washed with 0.5N HCl, brine, bicarbonate and brine again, and dried over magnesium sulfate. Evaporation of the solvent and recrystallization of the residue from dichloromethane/ther/hexane gives the product as an about 1/1 mixture of diastereomers.
MS (LC/MS): 593 (M+Na)
1 H-NMR (400MHz, CDCl3): 7.45-7.15 (m, 6H), 6.9-6.8 (m, 3H), 6.47/6.30 (d, 1 H), 5.90 (s, 1H), 5.08 (s, 2H), 5.0-4.85 (m, 1H), 4.1-3.98 (m, 2H), 3.77-3.70 (m, 1H), 3.3-3.15 (m, 2H), 2.95-2.85 (m, 2H), 2.6-2.5 (m, 1 H), 1.8-1.55 (m, 3H), 1.5-1.4 (m, 10H), 1.4-1.3 (m, 2H), 1.26/1.20 (d, 3H), 1.12-1.08 (m, 3H), 0.95-0.9 (m, 3H).

### g) {(S)-1-[(1S*,25*,4R*)-4-Butylcarbamoyl-2-hydroxy-1-(3-hydroxy-benzyl)-pentylcarbamoyl]-ethyl}-carbamic acid tert-butyl ester

{(S)-1-[(1S*,2S*,4R*)-1-(3-Benzyloxy-benzyl)-4-butylcarbamoyl-2-hydroxy-pentylcarbamoyl]-ethyl}-carbamic acid tert-butyl ester (4.44g, 7.8mmol) is hydrogenated at rt (1 atm H₂) in ethanol (240ml) with Pd/C (10% Engelhard 4505, 0.69g) for 4h. The catalyst is filtered off and the filtrate is evaporated to give a white foam (about 1/1 mixture of diastereomers).
MS (LC/MS): 502 (M+Na)
1 H-NMR (400MHz, CDCl3): 7.14 (t, 1 H), 6.78-6.7 (m, 3H), 6.65/6.55 (d, 1 H), 6.25/6.08 (s, 1 H), 5.26/5.08 (d, 1 H), 4.15-3.95 (m, 2H), 3.75-3.7 (m, 1 H), 3.3-3.15 (m, 2H), 2.93-2.8 (m, 2H), 2.65-2.52 (m, 1 H), 2.0-1.5 (m, 5H), 1.5-1.4 (m, 10H), 1.4-1.3 (m, 2H), 1.30/1.25 (d, 3H), 1.15-1.10 (m, 3H), 0.94 (t, 3H).

### h) 6-{3-[(2S*,3S*,5R*)-2-((S)-2-tert-Butoxycarbonylamino-propionylamino)-5-butylcarbamoyl-3-hydroxy-hexyl]-phenoxy}-hexanoic acid tert-butyl ester

{(S)-1-[(1S*,2S*,4R*)-4-Butylcarbamoyl-2-hydroxy-1-(3-hydroxy-benzyl)-pentylcarbamoyl]-ethyl}-carbamic acid tert-butyl ester (1.20g, 2.5mmol) is dissolved in acetone (250ml) and treated with 6-Bromo-hexanoic acid tert-butyl ester (CAS 65868-63-5, 0.94g, 3.8mmol, 1.5eq), water free potassium carbonate (1.04g, 7.5mmol, 3eq) and potassium iodide (0.42g, 2.5mmol, 1 eq). The mixture is heated at reflux temperature (bath temperature 75°C) for 5 days. The reaction mixture is diluted with EtOAc, washed with brine, dried over magnesium sulfate and evaporated. The crude product is purified by chromatography on silica (flashmaster, dichloromethane to dichloromethane/MeOH 90/10; mixed fractions are collected and resubmitted to chromatography). After a total of 5 chromatographies and recrystallization from ether/hexane white crystals are obtained (about 1/1 mixture of diastereomers).
MS (LC/MS): 672 (M+Na)
1 H-NMR (400MHz, C2D2Cl4): 7.25 (t, 1H), 6.88-6.82 (m, 3H), 6.32/6.20 (d, 1H), 5.33/5.17 (s, 1H), 4.43/4.33 (d, 1 H), 4.15-4.03 (m, 4H), 3.82 (s, 1 H), 3.45-3.2 (m, 3H), 3.03-2.90 (m, 2H), 2.60-2.53 (m, 1 H), 2.33 (t, 2H), 1.90-1.80 (m, 2H), 1.80-1.65 (m, 3H), 1.65-1.4 (m, 25H), 1.35/1.32 (d, 3H), 1.19 (d, 3H), 1.03 (t, 3H).

### i) (S)-1-{(1S,2S,4R)-4-Butylcarbamoyl-1-[3-(5-carboxy-pentyloxy)-benzyl]-2-hydroxypentylcarbamoyl}-ethyl-ammonium trifluoroacetate

6-{3-[(2S*,3S*,5R*)-2-((S)-2-tert-Butoxycarbonylamino-propionylamino)-5-butylcarbamoyl-3-hydroxy-hexyl]-phenoxyl-hexanoic acid tert-butyl ester (1.25g, 1.9mmol) is dissolved in trifluoroacetic acid (40ml) and water (4.5ml) at 0°C and stirred for 30min. The reaction mixture is diluted with 380ml cold water and the solvents are evaporated at rt. The two enantiomerically pure diastereomers are separated by preparative HPLC (Nucleosil 100-5 C18, water/acetonitrile 90/10 to acetonitrile). The first diastereomer is collected in about 80% purity and used directly for the next step, MS (LC/MS): 516 (M+Na).

### j) (2R,45)-N-Butyl-4-hydroxy-2-methyl-4-((10S,13S)-10-methyl-8,11-dioxo-2-oxa-9,12-diaza-bicyclo [13.3.1]nonadeca-1(18),15(19),16-trien-13-yl)-butyramide

(S)-1-{(1 S,2S,4R)-4-Butylcarbamoyl-1-[3-(5-carboxy-pentyloxy)-benzyl]-2-hydroxypentylcarbamoyl}-ethyl-ammonium trifluoroacetate (0.26g, 0.43mmol) is dissolved in DMF (60ml) and BOP (0.28g, 0.64mmol, 1:5eq) and diisopropylethylamine (0.47ml, 2.8mmol, 6.5eq) are added. The reaction mixture is stirred over night at rt. The solvent is evaporated at rt and the residue is taken into EtOAc. Washing with 0.5N HCl, brine, bicarbonate and brine again, drying over magnesium sulfate and evaporation of the solvent gives the crude product. This is purified by preparative HPLC (Nucleosil 100-5 C18, water/acetonitrile 90/10 to acetonitrile) to give the title compound as white powder after liophilisation.
MS (LC/MS): 498 (M+Na)
1H-NMR (400MHz, CD3OD): 7.15 (t, 1H), 6.8-6.7 (m, 3H), 4.53 (q, 1H), 4.15-4.05 (m, 2H), 4.05 (d, 1 H), 3.60 (dt, 1H), 3.18 (t, 2H), 2.86 (d, 1 H), 2.73-2.6 (m, 2H), 2.27-2.22 (m, 1H), 2.16-2.10 (m, 1H), 1.84-1.65 (m, 4H), 1.55-1.3 (m, 8H), 1.27 (d, 3H), 1.17 (d, 3H), 0.95 (t, 3H).

### Example 2: (2R*,4S*)-N-Butyl-4-hydroxy-2-methyl-4-((9S,12S*)-9-methyl-7,10-dioxo-2-oxa-8,11-diaza-bicyclo[12.3.1]octadeca-1(18),14,16-trien-12-yl)-butyramide

### a) 5-{3-[(2S*,3S*,5R*)-2-((S)-2-tert-Butoxycarbonytamino-propionylamino)-5-butylcarbamoyl-3-hydroxy-hexyl]-phenoxy}-pentanoic acid tert-butyl ester

{(S)-1-[(1S*,2S*,4R*)-4-Butylcarbamoyl-2-hydroxy-1-(3-hydroxy-benzyl)-pentylcarbamoyl]-ethyl}-carbamic acid tert-butyl ester (see example 1g, 1.30g, 2.7mmol) is dissolved in acetone (250ml) and treated with 5-bromo-pentanoic acid tert-butyl ester (CAS 88987-42-2, 0.96g, 4.1mmol, 1.5eq), water free potassium carbonate (1.12g, 8.1mmol, 3eq) and potassium iodide (0.45g, 2.7mmol, 1eq). The mixture is heated at reflux temperature (bath temperature 75°C) for 7 days. The reaction mixture is diluted with EtOAc, washed with brine, dried over magnesium sulfate and evaporated. The crude product is purified by chromatography on silica (flashmaster, dichloromethane to dichloromethane/MeOH 90/10; mixed fractions are collected and resubmitted to chromatography). After a total of 4 chromatographies a white foam is obtained (about 1/1 mixture of diastereomers).
MS (LC/MS): 658 (M+Na)
1 H-NMR (400MHz, CDCl3): 7.17 (t, 1 H), 6.79-6.70 (m, 3H), 6.52 (d, 1 H), 6.35 (d, 1 H), 5.94 (t, 1 H), 5.1-4.8 (m, 1 H), 4.1-3.93 (m, 4H), 3.75-3.7 (m, 1 H), 3.3-3.1 (m, 2H), 2.94-2.84 (m, 2H), 2.60-2.50 (m, 1H), 2.29 (t, 2H), 1.85-1.55 (m, 7H), 1.55-1.4 (m, 18H), 1.4-1.18 (m, 6H), 1.12-1.08 (m, 3H), 0.92 (t, 3H).

### b) (S)-1-{(1S*,2S*,4R*)-4-Butylcarbamoyl-1-[3-(4-carboxy-butoxy)-benzyl]-2-hydroxypentylcarbamoyl}-ethyl-ammonium trifluoroacetate

5-{3-[(2S*,3S*,5R*)-2-((S)-2-tert-Butoxycarbonylamino-propionylamino)-5-butylcarbamoyl-3-hydroxy-hexyl]-phenoxy}-pentanoic acid tert-butyl ester (1.46g, 2.3mmol) is dissolved in trifluoroacetic.acid (49.5ml) and water (5.5ml) at 0°C and stirred for 30min. The solvents are evaporated at rt. The residue is dissolved in dichloromethane and dried over sodium sulfate. Evaporation of the solvent yields a viscous oil that is used directly for the next step.

### c) (2R*,4S*)-N-Butyl-4-hydroxy-2-methyl-4-((9S,12S*)-9-methyl-7,10-dioxo-2-oxa-8,11-diaza-bicyclo[12.3.1]octadeca-1(18),14,16-trien-12-yl)-butyramide

(S)-1-{(1S*,2S*,4R*)-4-Butylcarbamoyl-1-[3-(4-carboxy-butoxy)-benzyl]-2-hydroxypentylcarbamoyl}-ethyl-ammonium trifluoroacetate (0.90g, 1.5mmol) is dissolved in DMF (210ml) and BOP (1.0g, 2.3mmol, 1.5eq) and diisopropylethylamine (1.69ml, 9.9mmol, 6.5eq) are added. The reaction mixture is stirred over night at rt. The solvent is evaporated at rt and the residue is taken into EtOAc. Washing with 0.5N HCl, brine, bicarbonate and brine again, drying over magnesium sulfate and evaporation of the solvent gives the crude product. This is purified by chromatography on silica (flashmaster, dichloromethane to dichloromethane/MeOH 90/10) followed by preparative HPLC (Nucleosil 100-5 C18, water/acetonitrile 90/10 to acetonitrile) to give the title compound as white powder after liophilisation (12mg, mixture of diastereomers).
MS (LC/MS): 484 (M+Na)
1 H-NMR (400MHz, DMSO): 8.04/7.85 (d, 1H), 7.93/7.42 (d, 1H), 7.60 (t, 1H), 7.10 (t, 1 H), 6.8-6.6 (m, 3H), 4:95/4.70 (d, 1 H), 4.2-4.0 (m, 2H), 3.95-3.35 (m, 3H), 3.05-2.9 (m, 2H), 2.85-2.55 (m, 2H), 2.4-2.3 (m, 2H), 2.25-2.05 (m, 1H), 1.75-1.45 (m, 5H), 1.45-0.9 (m, 10H), 0.87 (t, 3H).

### Example 3: (2R*,4S*)-N-Butyl-4-hydroxy-2-methyl-4-((8S,11S*)-8-methyl-6,9-dioxo-2-oxa-7,10-diaza-bicyclo[11.3.1]heptadeca-1(17),13,15-trien-11-yl)-butyramide

### a) 4-{3-[(2S*,3S*,5R*)-2-((S)-2-tert-Butoxycarbonylamino-propionylamino)-5-butytcarbamoyt-3-hydroxy-hexyl]-phenoxy}-butyric acid tert-butyl ester

{(S)-1-[(S*,2S*,4R*)-4-Butylcarbarnoyl-2-hydroxy-1-(3-hydroxy-benzyl)-pentylcarbamoyl]-ethyl}-carbamic acid tert-butyl ester (see example 1g, 1.30g, 2.7mmol) is dissolved in acetone (250ml) and treated with 5-Bromo-pentanoic acid tert-butyl ester (CAS 88987-42-2, 0.96g, 4.1mmol, 1.5eq), water free potassium carbonate (1.12g, 8.1mmol, 3eq) and potassium iodide (0.45g, 2.7mmol, 1eq). The mixture is heated at reflux temperature (bath temperature 75°C) for 7 days. The reaction mixture is diluted with EtOAc, washed with brine, dried over magnesium sulfate and evaporated. The crude product is purified by chromatography on silica (flashmaster, dichloromethane to dichloromethane/MeOH 90/10; mixed fractions are collected and resubmitted to chromatography). A white foam is obtained (about 1/1 mixture of diastereomers).
MS (LC/MS): 644 (M+Na)

### b) (S)-1-{(1S*,2S*,4R*)-4-Butylcarbamoyl-1-[3-(3-carboxy-propoxy)-benzyl]-2-hydroxypentylcarbamoyl}-ethyl-ammonium trifluoroacetate

4-{3-[(2S*,3S*,5R*)-2-((S)-2-tert-Butoxycarbonylamino-propionylamino)-5-butylcarbamoyl-3-hydroxy-hexyl]-phenoxy}-butyric acid tert-butyl ester (1.45g, 2.3mmol) is dissolved in trifluoroacetic acid (49.5ml) and water (5.5ml) at 0°C and stirred for 30min. The solvents are evaporated at rt. The residue is dissolved in dichloromethane and dried over sodium sulfate. Evaporation of the solvent yields a viscous oil that is used directly for the next step.
MS (LC/MS): 488 (M+Na)

### c) (2R*,4S*)-N-Butyl-4-hydroxy-2-methyl-4-((8S,11S*)-8-methyl-6,9-dioxo-2-oxa-7,10-diaza-bicyclo[11.3.1]octadeca-1(17),13,15-trien-11-yl)-butyramide

(S)-1-{(1 S*,2S*,4R*)-4-Butylcarbamoyl-1-[3-(3-carboxy-propoxy)-benzyl]-2-hydroxypentylcarbamoyl}-ethyl-ammonium trifluoroacetate (0.77g, 1.3mmol) is dissolved in DMF (180ml) and BOP (0.88g, 2.0mmol, 1.5eq) and diisopropylethylamine (1.47ml, 8.6mmol, 6.5eq) are added. The reaction mixture is stirred over night at rt. The solvent is evaporated at rt and the residue is taken into EtOAc. Washing with 0.5N HCl, brine, bicarbonate and brine again, drying over magnesium sulfate and evaporation of the solvent gives the crude product. This is purified by chromatography on silica (flashmaster, dichloromethane to dichloromethane/MeOH 90/10) followed by preparative HPLC (Nucleosil 100-5 C18, water/acetonitrile 90/10 to acetonitrile) to give the title compound as white powder after liophilisation (mixture of diastereomers).
MS (LC/MS): 470 (M+Na)
1H-NMR (400MHz, DMSO): 7.95-7.55 (m, 2H), 7.10-7.00 (m, 1 H), 6.8-6.6 (m, 3H), 4.7-4.5 (m, 1 H), 4.3-4.1 (m, 1 H), 3.95-3.85 (m, 1 H), 3.7-3.6 (m, 1H), 3.4-3.3 (m, 2H), 3.05-2.9 (m, 2H), 2.7-2.5 (m, 5H), 2.35-2.15 (m, 1 H), 2.0-1.65 (m, 3H), 1.45-1.15 (m, 6H), 1.05-0.95 (m, 4H), 0.87 (t, 3H).

### Example 4: (2R*,4S*)-N-Butyl-4-hydroxy-2-methyl-4-((11S*,14S*)-11-methyl-9,9,12-trioxo-2-oxa-9lambda*6*-thia-13-aza-bicyclo[14.3.1]icosa-1(20),16,18-trien-14-yl)-butyramide

### a) [(1S*,2S*,4R*)-4-Butylcarbamoyl-2-hydroxy-1-(3-hydroxy-benzyl)-pentyl]-carbamic acid tert-butyl ester

[(1S*,2S*,4R*)-1-(3-Benzyloxy-benzyl)-4-butylcarbamoyl-2-hydroxy-pentyl]-carbamic acid tert-butyl ester (see example 1e, 0.24g, 0.48mmol) is hydrogenated at rt (1 atm H₂) in ethanol (30ml) with Pd/C (10% Engelhard 4505, 0.060g) for 2h. The catalyst is filtered off and the filtrate is evaporated. The crude product is purified by chromatography on silica (flashmaster, dichloromethane to dichloromethane/MeOH 85/15) to give the product as a white foam; MS (LC/MS): 431 (M+Na).

### b) [(1S*,2S*,4R*)-1-(3-Allyloxy-benzyl)-4-butylcarbamoyl-2-hydroxy-pentyl]-carbamic acid tert-butyl ester

[(1S*,2S*,4R*)-4-Butylcarbamoyl-2-hydroxy-1-(3-hydroxy-benzyl)-pentyl]-carbamic acid tert-butyl ester (0.60g, 1.47mmol), water free potassium carbonate (0.61 g, 4.4mmol, 3eq), potassium iodide (0.244g, 1.47mmol, 1eq) and 3-bromo-propene (0.186ml, 2.2mmol, 1.5eq) are dissolved in dry acetone (150ml) and stirred at reflux temperature (bath temperature 75°C) during 3 days. The reaction mixture is diluted with EtOAc, washed with brine, dried over magnesium sulfate and the solvents are evaporated. Purification by chromatography on silica (flashmaster, dichloromethane to dichloromethane/MeOH 90/10) followed by recrystallization from ether/hexane gives the product as white solid.
MS (LC/MS): 471 (M+Na)

### c) (2R*,4S*,5S*)-6-(3-Allyloxy-phenyl)-4-hydroxy-2-methyl-5-[(S*)-2-(pent-4-ene-1-sulfonyl)-propionylamino]-hexanoic acid butylamide

[(1S*,2S*,4R*)-1-(3-Allyloxy-benzyl)-4-butylcarbamoyl-2-hydroxy-pentyl]-carbamic acid tert-butyl ester (0.26g, 0.58mmol) is dissolved in 4N hydrochloric acid in dioxane and stirred at rt for 1.5 h. The solvent is evaporated and the residue is dried at low pressure. The residue is redissolved in dry dichloromethane (20ml). Racemic 2-Methyl-3-(pent-4-ene-1-sulfonyl)-propionic acid (see intermediate 3, 0.165g, 0.75mmol, 1.2eq), EDCl (0.179g, 0.94mmol, 1.5eq), HOBt (0.115g, 0.75mmol, 1.2eq) and triethylamine (0.26ml, 1.87mmol, 3eq) are added. The reaction mixture is stirred under argon at rt for 18 h. The mixture is diluted with EtOAc, washed with 0.5N aqueous HCl, brine, sodium bicarbonate solution and again with brine. Drying over magnesium sulfate and evaporation of the solvents gives the crude product (mixture of two racemic diastereomers). The diastereomeres are separated by chromatography on silica (flashmaster, EtOAc/hexanes 1/1 to EtOAc), mixed fractions are collected and separated again on a preparative TLC plate to give the desired racemic diastereomer (eluted as second compound, Rf = 0.47 in EtOAc).
MS (LC/MS): 573 (M+Na)

### d) (2R*,4S*)-N-Butyl-4-hydroxy-2-methyl-4-((11.S*,14S*)-11-methyl-9,9,12-trioxo-2-oxa-9lambda*6*-thia-13-aza-bicyclo[14.3.1]icosa-1(20),16,18-trien-14-yl)-butyramide

(2R*,4S*,5S*)-6-(3-Allyloxy-phenyl)-4-hydroxy-2-methyl-5-,[(S*)-2-(pent-4-ene-1-sulfonyl)-propionylamino]-hexanoic acid butylamide (0.115g, 0.21mmol) is dissolved in dry dichloromethane (220ml) under argon and benzylidene(1,3-dimesitylimidazolidin-2-ylidene)(tricyclohexy)phosphine)ruthenium dichloride (CAS 246047-72-3) (9mg, 5mol%) is added. The reaction is stirred at reflux temperature (bath temperature 60°C) for 2.5 h. The solvent is evaporated and the residue is purified by chromatography on silica (flashmaster, EtOAc/hexanes 1/1 to EtOAc). The resulting off-white solid is hydrogenated with Pd/C (10% Engelhard 4505, 0.020g) in methanol (3ml) at rt at 1 atm hydrogen during 3 h. After filtering through glass wool the solvent is evaporated and the crude product is purified on a preparative TLC plate (dichloromethane/MeOH 95/5). Recrystallization from dichloromethane/ether/hexane gives the product.
MS (LC/MS): 547 (M+Na)
1 H-NMR (400MHz, CDCl3): 7.23 (t, 1 H), 6.84-6.76 (m, 3H), 6.15 (d, 1 H), 5.88 (t, 1 H), 4.31 (t, 1 H), 4.18-4.10 (m, 2H), 3.90 (d, 1 H), 3.33-3.18 (m, 3H), 3.01-2.84 (m, 3H), 2.71-2.54 (m, 4H), 1.85-1.60 (m, 7H), 1.60-1.45 (m, 6H), 1.45-1.32 (m, 2H), 1.31 (d, 3H), 1.28 (d, 3H), 0.96 (t, 3H).

### Example 5: (2R,4S)-N-Butyl-4-hydroxy-2-methyl-4-((9S,12S)-9-methyl-7,7,10-trioxo-2-oxa-7lambda*6*-thia-11-aza-bicyclo[12.3.1]octadeca-1(18),14,16-trien-12-yl)-butyramide

### a) (2R*,4S*,5S*)-6-(3-Allyloxy-phenyl)-4-hydroxy-2-methyl-5[(S)-2-methyl-3-(prop-2-ene-1-sulfonyl)-propionylamino]-hexanoic acid butylamide

[(1 S*,2S*,4R*)-1-(3-Allyloxy-benzyl)-4-butylcarbamoyl-2-hydroxy-pentyl]-carbamic acid tert-butyl ester (see example 4b, 0.275g, 0.61 mmol) is dissolved in 4N hydrochloric acid in dioxane and stirred at rt for 1.5 h. The solvent is evaporated and the residue is dried at low pressure. The residue is redissolved in dry dichloromethane (20ml). (S)-2-Methyl-3-(prop-2-ene-1-sulfonyl)-propionic acid (see intermediate 2, 0.138g, 0.72mmol, 1.2eq), EDCl (0.172g, 0.90mmol, 1.5eq), HOBt (0.110g, 0.72mmol, 1.2eq) and triethylamine (0.25ml, 1.8mmol, 3eq) are added. The reaction mixture is stirred under argon at rt for 18 h. The mixture is diluted with EtOAc, washed with 0.5N aqueous HCl, brine, sodium bicarbonate solution and again with brine. Drying over magnesium sulfate, evaporation of the solvents and purification by chromatography on silica (flashmaster, dichloromethane to dichloromethane/MeOH 95/5) gives the product as a 1:1 mixture of two enatiomerically pure diastereomers.
MS (LC/MS): 545 (M+Na)
1 H-NMR (400MHz, CDCl3): 7.23-7.18 (m, 1 H), 6.87-6.77 (m, 3H), 6.25-5.80 (m, 5H), 5.55-5.42 (m, 3H), 5.31 (d, 1 H), 4.55 (d, 2H), 4.4-4.0 (m, 3H), 3.82-3.42 (m, 4H), 3.35-3.17 (m, 2H), 2.97-2.75 (m, 4H), 2.60-2.53 (m, 1H), 1.83-1.60 (m, 3H), 1.53-1.46 (m, 2H), 1.41-1.29 (m, 2H), 1.32/1.17 (d, 3H), 1.13/0.95 (t, 3H).

### b) (2R,4S)-N-Butyl-4-hydroxy-2-methyl-4-((9S,12S)-9-methyl-7,7,10-trioxo-2-oxa-7lambda*6*-thia-11-aza-bicyclo[12.3.1]octadeca-1(18),14,16-trien-12-yl)-butyramide

(2R*,4S*,5S*)-6-(3-Allyloxy-phenyl)-4-hydroxy-2-methyl-5-[(S)-2-methyl-3-(prop-2-ene-1-sulfonyl)-propionylamino]-hexanoic acid butylamide (0.230g, 0.44mmol) is dissolved in dry dichloromethane (440ml) under argon and benzylidene(1,3-dimesitylimidazolidin-2-ylidene)(tricyclohexylphosphine)ruthenium dichloride (CAS 246047-72-3) (19mg, 5mol%) is added. The reaction is stirred at reflux temperature (bath temperature 60°C) for 3 h. The solvent is evaporated and the residue is purified by chromatography on silica (flashmaster, EtOAc/hexanes 1/1 to EtOAc) followed by a second chromatography on silica (flashmaster, dichloromethane to dichloromethane/MeOH 90/10). The resulting solid is recrystallized from dichloromethane/ether/hexane and then hydrogenated with Pd/C (10% Engelhard 4505, 0.035g) in methanol (5ml) at rt at 1 atm hydrogen during 3 h. After filtering through glass wool the solvent is evaporated and the two diastereomers are separated by preparative TLC (dichloromethane/MeOH 95/5). Recrystallization of the more polar diastereomer from dichloromethane/ether/hexane gives the product;MS (LC/MS): 519 (M+Na).
1 H-NMR (400Mhiz, C2D2Cl4): 7.29 (t, 1 H), 6.9-6.8 (m, 3H), 5.70 (d, 1 H), 5.66 (t, 1H), 4.35-4.18 (m, 3H), 3.95-3.85 (m, 1 H), 3.64 (dd, 1 H), 3.30 (q, 2H), 3.05-2.8 (m, 5H), 2.68-2.57 (m, 2H), 2.1-1.65 (m, 6H), 1.6-1.4 (m, 4H), 1.37 (d, 3H), 1.30 (d, 3H), 1.02 (t, 3H).

### Example 6: (2R,4S)-N-Butyl-4-hydroxy-2-methyl-4-((10S,13S)-10-methyl-8,8,11-trioxo-2-oxa-8lambda*6*-thia-12-aza-bicyclo[13.3.1]nonadeca-1(19),15,17-trien-13-yl)-butyramide

### a) (2R,4S,5S)-6-(3-Allyloxy-phenyl)-5-[(S)-3-(but-3-ene-1-sulfonyl)-2-methyl-propionylamino]-4-hydroxy-2-methyl-hexanoic acid butylamide

The enantiomers of [(S*)-2-(3-Benzyloxy-phenyl)-1-((2S*,4R*)-4-methyl-5-oxo-tetrahydrofuran-2-yl)-ethyl]-carbamic acid tert-butyl ester (example 1d) are separated by preparative chiral HPLC to give [(S)-2-(3-benzyloxy-phenyl)-1-((2S,4R)-4-methyl-5-oxo-tetrahydro-furan-2-yl)-ethyl]-carbamic acid tert-butyl ester (α_{D} = +10°, c=1 in CHCl3). Derivatization according to example 4a-4b gives [(1S,2S,4R)-1-(3-allyloxy-benzyl)-4-butylcarbamoyl-2-hydroxypentyl]-carbamic acid tert-butyl ester.
[(1S,2S,4R)-1-(3-allyloxy-benzyl)-4-butylcarbamoyl-2-hydroxy-pentyl]-carbamic acid tert-butyl ester (0.29g, 0.58mmol) is dissolved in 4N hydrochloric acid in dioxane and stirred at rt for 1.5 h. The solvent is evaporated and the residue is dried at low pressure. The residue is redissolved in dry dichloromethane (20ml). (S)-3-(But-3-ene-1-sulfonyl)-2-methyl-propionic acid (see intermediate 1, 0.158g, 0.76mmol, 1.2eq), EDCI (0.183g, 0.96mmol,1.5eq), HOBt (0.117g, 0.76mmol, 1.2eq) and triethylamine (0.27ml, 1.90mmol, 3eq) are added. The reaction mixture is stirred under argon at rt for 18 h. The mixture is diluted with EtOAc, washed with 0.5N aqueous HCl, brine, sodium bicarbonate solution and again with brine.

Drying over magnesium sulfate and evaporation of the solvents gives the crude product that is purified by chromatography on silica (flashmaster, EtOAc/hexanes 1/1 to EtOAc). Recrystallization from dichloromethane/ether/hexane gives the product.
MS (LC/MS): 559 (M+Na)

### b) (2R,4S)-N-Butyl-4-hydroxy-2-methyl-4-((10S,13S)-10-methyl-8,8,11-trioxo-2-oxa-8lambda*6*-thia-1 2-aza-bicyclo[1 3.3.1]nonadeca-1(19),15,17-trien-13-yl)-butyramide

(2R,4S,5S)-6-(3-Allyloxy-phenyl)-5-[(S)-3-(but-3-ene-1-sulfonyl)-2-methyl-propionylamino]-4-hydroxy-2-methyl-hexanoic acid butylamide (0.220g, 0.41mmol) is dissolved in dry dichloromethane (430ml) under argon and benzylidene(1,3-dimesitylimidazolidin-2-ylidene)(tricyclohexylphosphine)ruthenium dichloride (CAS 246047-72-3) (17mg, 5mol%) is added. The reaction is stirred at reflux temperature (bath temperature 60°C) for 2.5 h. The solvent is evaporated and the residue is purified by chromatography on silica (flashmaster, EtOAc/hexanes 1/1 to EtOAc) followed by a second chromatography on silica (flashmaster, dichloromethane to dichloromethane/MeOH 90/10). The resulting solid is recrystallized from dichloromethane/etherlhexane and then hydrogenated with Pd/C (10% Engelhard 4505, 0.020g) in methanol (5ml) at rt at 1 atm hydrogen during 4 h. After filtering through glass wool the solvent is evaporated and the product is purified by preparative TLC (dichloromethane/MeOH 95/5) followed by recrystallization from dichloromethane/ether/hexane to give the product; MS (LC/MS): 533 (M+Na).
1 H-NMR (400MHz, C₂D₂Cl₄): 7.29 (t, 1H), 6.37 (d, 1 H), 6.3-6.25 (m, 2H), 5.84 (d, 1 H), 5.66 (s, 1 H), 4.25-4.15 (m, 3H), 3.90 (d, 1H), 3.80 (s, 1 H), 3.35-3.25 (m, 3H), 2.98 (dd, 1 H), 2.95-2.75 (m, 4H), 2.70 (dd, 1H), 2.65-2.55 (m, 1 H), 2.0-1.6 (m, 8H), 1.6-1.5 (m, 2H), 1.5-1.35 (m, 2), 1.38 (d, 3H), 1.30 (d, 3H), 1.00 (t, 3H).

### Example 7: (2R*,4S*)-N-Butyl-4-hydroxy-2-methyl-4-((S*)-7,7,10-trioxo-2-oxa-7lambda*6*-thia-11-aza-bicyclo[12.3.1]octadeca-1(18),14,16-trien-12-yl)-butyramide

### a) (2R*,4S*,5S*)-6-(3-Allyloxy-phenyl)-4-hydroxy-2-methyl-5-[3-(prop-2-ene-1-sulfonyl)-propionylamino]-hexanoic acid butylamide

[(1S*,25*,4R*)-1-(3-Allyloxy-benzyl)-4-butylcarbamoyl-2-hydroxy-pentyl]-carbamic acid tert-butyl ester (see example 4b, 0.700g, 1.56mmol) is dissolved in 4N hydrochloric acid in dioxane and stirred at rt for 2.5 h. The solvent is evaporated and the residue is dried at low pressure. The residue is redissolved in dry dichloromethane (20ml). 3-(Prop-2-ene-1-sulfonyl)-propionic acid (see intermediate 4, 0.333g, 1.87mmol, 1.2eq), EDCI (0.448g, 2.33 mmol, 1.5eq), HOBt (0.286g, 1.87 mmol, 1.2eq) and triethylamine (0.65ml, 4.7mmol, 3eq) are added. The reaction mixture is stirred under argon at rt for 18 h. The mixture is diluted with EtOAc, washed with 0.5N aqueous HCl, sodium bicarbonate solution and brine. Drying over magnesium sulfate, evaporation of the solvents and purification by flash-chromatography on silica (dichloromethane to dichloromethane/MeOH 93/7) gives the product as white solid; MS (LC/MS): 531 (M+Na).
1H-NMR (400MHz, CDCl3): 7.18 (t, 1H), 6.82-6.72 (m, 3H), 6.16 (d, 1H), 6.10-5.82 (m, 3H), 5.53-5.37 (m, 3H), 5.27 (d, 1 H), 4.53 (d, 2H), 4.44 (d, 1 H), 4.06 (q, 1 H), 3.78-3.63 (m, 3H), 3.38-3.15 (m, 4H), 2.94-2.83 (m, 2H), 2.76-2.47 (m, 3H), 1.53-1.43 (m, 2H), 1.40-1.30 (m, 2H), 1.13 (d, 3H), 0.94 (t, 3H).

### b) (2R*,4S*)-N-Butyl-4-hydroxy-2-methyl-4-((S*)-7,7,10-trioxo-2-oxa-7lambda*6*-thia-11-aza-bicyclo[12.3.1]octadeca-1(18),14,16-trien-12-yl)-butyramide

(2R*,4S*,5S*)-6-(3-Allyloxy-phenyl)-4-hydroxy-2-methyl-5-[3-(prop-2-ene-1-sulfonyl)-propionylamino]-hexanoic acid butylamide (0.370g, 0.73mmol) is dissolved in dry dichloromethane (400ml) under argon and benzylidene(1,3-dimesitylimidazolidin-2-ylidene)(tricyclohexylphosphine)ruthenium dichloride (CAS 246047-72-3) (31 mg, 5mol%) is added. The reaction is stirred at reflux temperature (bath temperature 60°C) for 6 h. The solvent is evaporated and the residue is purified by chromatography on silica (dichloromethane to dichloromethane/MeOH 90/10). The resulting solid is hydrogenated with Pd/C (0.050g, 10% Engelhard 4505) in methanol (30ml) at rt at 1 atm hydrogen for 19h.
After filtering through glass wool the solvent is evaporated. The residue is dissolved in ethanol and treated with charcoal. Filtration over celite and evaporation gives the product;
MS (LC/MS): 505 (M+Na).
1 H-NMR (400MHz, DMSO): 7.82 (d, 1 H), 7.68 (t, 1 H), 7.13 (t, 1H), 6.8-6.7 (m, 3H), 4.88 (s, 1H), 4.2-4.05 (m, 2H), 3.85 (t, 1 H), 3.42-3.25 (m, 4H), 3.06-2.7 (m, 5H), 2.65-2.35 (m, 3H), 1.85-1.6 (m, 6H), 1.4-1.15 (m, 4H), 1.00 (d, 3H), 0.87 (t, 3H).

### Example 8: (2R,4S)-N-Butyl-4-hydroxy-2-methyl-4-((3S,6S)-6-methyl-5,8,8-trioxo-8lambda*6*-thia-4-aza-bicyclo[12.3.1]octadeca-1(17),14(18),15-trien-3-yl)-butyramide

### a) [(S*)-2-(3-Hydroxy-phenyl)-1-((2S*,4R*)-4-methyl-5-oxo-tetrahydro-furan-2-yl)-ethyl]-carbamic acid tert-butyl ester

[(S*)-2-(3-Benzyloxy-phenyl)-1-((2S*,4R*)-4-methyl-5-oxo-tetrahydro-furan-2-yl)-ethyl]-carbamic acid tert-butyl ester (see example 1d, 1.02g, 2.4mmol) is hydrogenated (1atm H2) in ethanol (75ml) at rt with Pd/C (10% Engelhard 4505, 0.2g) for 0.5h. Filtration through a bed of hyflo and evaporation of the solvent gives the product as a white foam.
MS (LC/MS): 358 (M+Na)

### b) Trifluoro-methanesulfonic acid 3-[(S*)-2-tert-butoxycarbonytamino-2-((2S*,4R*)-4-methyl-5-oxo-tetrahydro-furan-2-yl)-ethyl]-phenyl ester

[(S*)-2-(3-Hydroxy-phenyl)-1-((2S*,4R*)-4-methyl-5-oxo-tetrahydro-furan-2-yl)-ethyl]-, carbamic acid tert-butyl ester (0.95g, 2.8mmol), N-phenyl-bis-(trifluoromethanesulfnimide) (CAS 37595-74-7, 1.01g, 2.8mmol, 1.0eq) and waterfree potassium carbonate (1.17g, 8.5mmol, 3eq) in 10ml dry THF are heated in a microwave apparatus at 120°C for 1.5h. The mixture is diluted with EtOAc and washed with brine. Drying over magnesium sulfate and evaporation of the solvent followed by purification by chromatography on silica (flashmaster, hexane to hexane/EtOAc 60/40) gives the product;MS (LC/MS): 490 (M+Na).
1 H-NMR (400MHz, CDCl3): 7.44 (t, 1 H), 7.32 (d, 1 H), 7.21-7,19 (m, 2H), 4.57 (d, 1 H), 4.52 (t, 1 H), 4.06 (q, 1 H), 2.99 (d, 2H), 2.81-2.71 (m, 1H), 2.44-2.38 (m, 1 H), 1.97-1.90 (m, 1 H), 1.40 (s, 9H), 1.31 (d, 3H).

### c) [(S*)-2-(3-Allyl-phenyl)-l-((2S*,4R*)-4-methyl-5-oxo-tetrallydro-furan-2-yl)-ethyl]-carbamic acid tert-butyl ester

Trifluoro-methanesulfonic acid 3-[(S*)-2-tert-butoxycarbonylamino-2-((2S*,4R*)-4-rnethyl-5-oxo-tetrahydro-furan-2-yl)-ethyl]-phenyl ester (1.01g, 2.16mmol) is dissolved in dry DMF. Under argon but-3-enyl-tributyl-stannane (0.75ml, 2.38mmol, 1.1eq), waterfree lithiumchloride (0.23g, 5.4mmol, 2.5eq) and bis-(triphenylphosphin)-palladium(II)-chloride (30mg, 0.04mmol, 1.9mol%) are added and the mixture is stirred at 100°C bath temperature for 45min. After cooling to rt the mixture is diluted with EtOAc, washed with brine, dried over magnesium sulfate and the solvents are evaporated. The residue is purified by chromatography on silica (flashmaster, hexane to hexane/EtOAc 50/50) followed by crystallization from ether/hexane to give the product as white crystals.
MS (LC/MS): 382 (M+Na)

### d) (S)-N-[(S)-2-(3-Allyl-phenyl)-1-((2S,4R)-4-methyl-5-oxo-tetrahydro-furan-2-yl)-ethyl] - 3-(but-3-ene-1-sulfonyl)-2-methyl-propionamide

[(S*)-2-(3-Allyl-phenyl)-1-((2S*,4R*)-4-methyl-5-oxo-tetrahydro-furan-2-yl)-ethyl]-carbamic acid tert-butyl ester (0.32g, 1.39mmol) is dissolved in 4N hydrochloride in dioxane (8ml) and stirred at rt for 1.5h. The sovent is evaporated and the residue is redissolved in dry dichloromethane (20ml). (S)-3-(But-3-ene-1-sulfonyl)-2-methyl-propionic acid (see intermediate 1, 0.209g, 10mmol, 1.2eq), EDCl (0.243g, 1.27mmol, 1.5eq), HOBt (0.155g, 10mmol, 1.2eq) and triethylamine (0.35ml, 2.5mmol, 3eq) are added and the reaction mixture is stirred under argon at rt for 18h. The mixture is diluted with EtOAc, washed with 0.5N aqueous hydrochloric acid, sodium bicarbonate solution and brine, dried over magnesium sulfate and the solvents are evaporated. The two enatiomerically pure diastereomers are separated by chromatography on silica (flashmaster, hexane to hexane/EtOAc 50/50). Mixted fractions are collected and resubmitted to chromatography. The desired compound is the more polar of the two diastereomers (Rf = 0.4 in hexane/EtOAc 3/7) and is obtained as yellowish solid; MS (LC/MS): 470 (M+Na);
1H-NMR (400MHz, CDCl3): 7.25 (t, 1 H), 7.13-7.07 (m, 3H), 6.18 (d, 1 H), 6.02-5.92 (M, 1 H), 5.85-5.75 (m, 1 H), 5.20-5.06 (m, 4H), 4.60-4.53 (m, 1 H), 4.37 (q, 1H), 3.43-3.35 (m, 3H), 3.03-2.83 (m, 6H), 2.73-2.64 (m, 1H), 2.60-2.53 (m, 2H), 2.32-2.25 (m, 1 H), 1.93-1.86 (m, 1 H), 1.33 (d, 3H), 1.27 (d, 3H).

### e) (2R,4S)-N-Butyl-4-hydroxy-2-methyl-4-((3S,6S)-6-methyl-5,8,8-trioxo-8lambda*6*-thia-4-aza-bicyclo[12.3.1]octadeca-1(17),14(18),15-trien-3-yl)-butyramide

(S)-N-[(S)-2-(3-Allyl-phenyl)-1-((2S,4R)-4-methyl-5-oxo-tetrahydro-furan-2-yl)-ethyl]-3-(but-3-ene-1-sulfonyl)-2-methyl-propionamide (140mg, 0.31 mmol) is dissolved in dry dichloromethane under argon and benzylidene(1,3-dimesitylimidazolidin-2-ylidene)(tricyclohexylphosphine)ruthenium dichloride (CAS 246047-72-3) (13mg, 5mol%) is added. The reaction is heated at reflux temperature (bath temperature 60°C) for 2.5h. The solvent is evaporated and the residue is purified by chromatography on silica (flashmaster, hexane/EtOAc 80/20 to EtOAc) to give the ring-closed intermediate (3S,6S)-6-Methyl-3-((2S,4R)-4-methyl-5-oxo-tetrahydro-furan-2-yl)-8,8-dioxo-8lambda*6*-thia-4-azabicyclo[12.3.1]octadeca-1(18),11,14,16-tetraen-5-one as a grayish solid (98mg, 0.23mmol). This is dissolved in butylamine (5ml) and stirred for 18h at reflux temperature (bath temperature 90°C). The solvent is evaporated and the residue is purified by chromatography on silica (flashmaster, dichloromethane to dichloromethane/MeOH 92/8) and crystallization from dichloromethane/etherlhexane to give 58mg white crystals. They are dissolved in methanol (5ml) and hydrogenated (1atm H2) at rt with Pd/C (10% Engelhard 4505, 30mg) for 5h. Purification by chromatography on silica (flashmaster, dichloromethane to dichloromethane/MeOH 90/10) and crystallization from dichloromethane/ether/hexane gives the product as white crystals; MS (LC/MS): 517 (M+Na);
1 H-NMR (400MHz, CDCl3): 7.26 (t, 1 H), 7.10-7.06 (m, 3H), 5.96 (d, 1 H), 5.81 (t, 1 H), 4.39 (t, 1 H), 3.95-3.90 (m, 1 H), 3.35-3.25 (m, 3H), 3.03-2.68 (m, 2H), 2.8-2.4 (m, 7H), 1.8-1.45 (m, 9H), 1.45-1.25 (m, 4H), 1.32 (d, 3H), 1.30 (d, 3H), 0.96 (t, 3H).

### Example 9: (2R,4S)-N-Butyl-4-hydroxy-2-methyl-4-((3S,6S)-6-methyl-5,8,8-trioxo-8lambda*6*-thia-4-aza-bicyclo[13.3.1]nonadeca-1(18),15(19),16-trien-3-yl)-butyramide

The title compound is obtained in analogues manner as described for example 8, starting from [(S*)-2-(3-Allyl-phenyl)-1-((2S*,4R*)-4-methyl-5-oxo-tetrahydro-furan-2-yl)-ethyl]-carbamic acid tert-butyl ester (see example 8c) and (S)-2-methyl-3-(pent-4-ene-1-sulfonyl)-propionic acid (see intermediate 3a); MS (LC/MS): 531 (M+Na),
1 H-NMR (400MHz, CDCl3): 7.24 (t, 1 H), 7.10-7.04 (m, 3H), 6.00 (d, 1 H), 5.84 (t, 1H), 4.37 (t, 1 H), 3.95-3.87 (m, 1 H), 3.61 (dd, 1H), 3.31-3.26 (m, 2H), 3.02 (d, 1H), 2.92-2.85 (m, 1H), 2.82-2.76 (m, 1 H), 2.68-2.55 (m, 4H), 2.34-2.22 (m, 2H), 1.78-1.58 (m, 6H), 1.56-1.48 (m, 2H), 1.43-1.16 (m, 7H), 1.34 (d, 3H), 1.29 (d, 3H), 0.96 (t, 3H).

### Intermediate 1: (S)-3-(But-3-ene-1-sulfonyl)-2-methyl-propionic acid

### a) (S)-3-But-3-enylsulfanyl-2-methyl-propionic acid

(S)-3-Acetylsulfanyl-2-methyl-propionic acid (5.0ml, 36mmol) and 4-bromo-but-1-ene (3.75ml, 36mmol, 1.0eq) are dissolved in MeOH (55ml) and 4 N sodiumhydroxide in water (27.2ml, 108mmol, 3eq) are added under initial cooling. The mixture is stirred at rt for 2 h. Aqueous 1N hydrochloric acid (90ml) is added and the mixture is extracted with EtOAc. Washing with water and brine, drying over magnesium sulfate and evaporation of the solvent gives the crude product that is purified by chromatography on silica (flashmaster, hexane to hexane/EtOAc 40/60). The product is obtained as yellowish oil.

### b(S)-3-(But-3-ene-1-sulfonyl)-2-methyl-propionic acid

(S)-3-But-3-enylsulfanyl-2-methyl-propionic acid (1.0g, 5.74mmol) is dissolved in water (5ml) and acetonitrile (10ml) and cooled to -10°C. Oxone (3.49g 47%, 23mmol, 4eq) is added in portions, keeping the temperature below 0°C. The reaction is then stirred without cooling for 36h. The mixture is diluted with water and extracted with EtOAc. The organic layer is washed with water and brine, dried over magnesium sulfate and the solvent is evaporated. The crude product is purified by chromatography on silica (flashmaster, dichloromethane to dichloromethane/MeOH 90/10). Tituration with cold hexane gives the product as white crystals; MS (LC/MS): 229 (M+Na).

### Intermediate 2: (S)-2-Methyl-3-(prop-2-ene-1-sulfonyl)-propionic acid

(S)-2-Methyl-3-(prop-2-ene-1-sulfonyl)-propionic acid is obtained in analogous manner as described for intermediate 1, starting from (S)-3-acetylsulfanyl-2-methyl-propionic acid and 3-bromo-prop-1-ene.

### Intermediate 3: 2-Methyl-3-(pent-4-ene-1-sulfonyl)-propionic acid

2-Methyl-3-(pent-4-ene-1-sulfonyl)-propionic acid is obtained in analogous manner as described for intermediate 1, starting from 3-acetylsulfanyl-2-methyl-propionic acid and 5-bromo-pent-1-ene.

### Intermediate 3a: (S)-2-Methyl-3-(pent-4-ene-1-sulfonyl)-propionic acid

(S)-2-Methyl-3-(pent-4-ene-1-sulfonyl)-propionic acid is obtained in analogous manner as described for intermediate 1, starting from (S)-3-acetylsulfanyl-2-methyl-propionic acid and 5-bromo-pent-1-ene.

### Intermediate 4: 3-(Prop-2-ene-1-sulfonyl)-propionic acid

### a) 3-Allylsulfanyl-propionic acid

3-Mercapto-propionic acid (10.0 ml, 115mmol) and 3-bromo-propene (9.72ml, 115mmol, 1.0eq) are dissolved in methanol (28ml) and cooled in an icebath. 4N aqueous sodium hydroxid solution (86.2ml, 0.34mmol, 3eq) is added and the mixture is stirred at rt for 6h. 1N aqueous hydrochloric acid is added until acidic pH and the mixture is extracted with EtOAc. Washing with water and brine, drying over magnesium sulfate and evaporation of the solvent gives the product as a yellow oil.

### b) 3-(Prop-2-ene-1-sulfonyl)-propionic acid

3-Allylsulfanyl-propionic acid (0.94g, 6.4mmol) is dissolved in water (10ml) and acetonitrile (20ml) and cooled to -10°C. Oxone (19.8g 47%, 32mmol, 5eq) is added in portions, keeping the temperature below 0°C. The reaction is then stirred without cooling for 8h. The precipitate is filtered off and washed with acetonitrile. The filtrate is evaporated at reduced pressure and then distributed between water and EtOAc. The organic layer is washed with water and brine and dried over magnesium sulfate. Evaporation of the solvent gives the product as a white solid.

## Claims

1. A compound of the formula 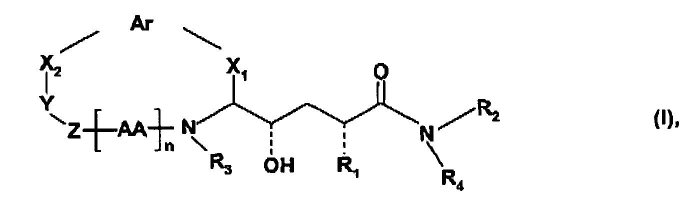 wherein
R₁ is (C₁₋₈)alkyl, (C₁₋₄)alkoxy(C₁₋₄)alkyl, hydroxy(C₁₋₈)alkyl, (C₁₋₄)alkylthio(C₁₋₄)alkyl, (C₁₋₆)alkenyl, (C₃₋₇)cycloalkyl, (C₃₋₇)cycloalkyl(C₁₋₄)alkyl, piperidinyl or pyrrolidinyl,
R₂ and R₄, independently, are hydrogen or optionally substituted (C₁₋₈)alkyl, (C₃₋₇)-cycloalkyl, (C₃₋₇)cycloalkyl(C₁₋₄)alkyl, aryl, aryl(C₁₋₄)alkyl, heteroaryl or heteroaryl(C₁₋₄) alkyl, or
R₂ and R₄, together with the nitrogen atom, to which they are attached, form an optionally substituted piperidinyl, pyrrolidinyl, morpholinyl or piperazinyl group,
R₃ is hydrogen or (C₁₋₄)alkyl,
X₁ is CH₂,
X₂ is CH₂, O, S, CO, COO, OCO, NHCO, CONH or NR, R being hydrogen or (C₁₋₄)alkyl,
Y is (C₁₋₈)alkylene. (C₁₋₈)alkylenoxy(C₁₋₆)alkylene, (C₁₋₈)alkenylene or (C₁₋₈)alkenylenoxy(C₁₋₆)alkylene,
Ar is a phenyl ring optionally mono-, di- or trisubstituted by, independently, hydroxy or halogen, whereby X₁ and X₂ are in meta or para position to each other,
and either
Z is CO,
AA is a natural or unnatural alpha-amino-acid, and
n is 0 or 1,
or
Z is SO₂
AA is an optionally substituted ethylenecarbonyl group (derived from a natural or unnatural alpha-amino acid by replacement of N-H in the alpha-amino group by a methylene group), and
n is 1,
in free base form or in acid addition salt form.

2. A compound according to claim 1 of the formula I, wherein
R₁ is (C₁₋₈)alkyl, (C₁₋₄)alkoxy(C₁₋₄)alkyl, hydroxy(C₁₋₆)alkyl, (C₁₋₄)alkylthio(C₁₋₄)alkyl, (C₁₋₆)alkenyl, (C₃₋₇)cycloalkyl, (C₃₋₇)cycloalkyl(C₁₋₄)alkyl, piperidinyl or pyrrolidinyl,
R₂ and R₄, independently, are
(a) hydrogen,
(b) (C₁₋₈)alkyl, (C₃₋₇)cycloalkyl or (C₃₋₇)cycloalkyl(C₁₋₄)alkyl, in each case optionally substituted by one to three groups selected from hydroxy, hydroxy(C₁₋₄)alkyl, (C₁₋₄)alkoxy, (C₁₋₄)alkoxy(C₁₋₄)alkyl, (C₁₋₄)alkoxy(C₁₋₄)alkoxy, (C₁₋₄)alkylsulfanyl, (C₁₋₄)alkoxycarbonyl, (C₁₋₄)alkylcarbonyloxy, (C₁₋₄)alkylcarbonylamino, (C₁₋₄)-alkylcarbonyl, cyano, oxo, hetero(C₃₋₇)cycloalkyl or heteroaryl or
(c) aryl, aryl(C₁₋₄)alkyl, heteroaryl or heteroaryl(C₁₋₄)alkyl, wherein in the latter two radicals heteroaryl denotes an aromatic 5- or 6- membered ring, in which 1, 2 or 3 atoms are heteroatoms independently selected from O, N and S, wherein all radicals are optionally substituted by one to three groups selected from halogen, hydroxy, cyano, trifluoromethyl, carboxy, (C₁₋₄)alkyloxycarbonyl, (C₁₋₄)alkyl-carbamoyl, (C₁₋₄)alkylsulfonyl, (C₁₋₄)alkylcarbonyloxy, (C₁₋₄)alkylcarbonyl, (C₁₋₄)alkyl, (C₁₋₄)alkoxy or hydroxy(C₁₋₄)alkyl, or
R₂ and R₄, together with the nitrogen atom, to which they are attached, form a piperidinyl, pyrrolidinyl, morpholinyl or piperazinyl group, each of which is optionally substituted by one to three groups selected from hydroxy, hydroxy-(C₁₋₄)alkyl, (C₁₋₄)alkoxy, (C₁₋₄)alkoxy(C₁₋₄)alkyl, (C₁₋₄)alkoxy(C₁₋₄)alkoxy, (C₁₋₄)-alkylsulfanyl, (C₁₋₄)alkoxycarbonyl, (C₁₋₄)alkylcarbonyloxy, (C₁₋₄)alkylcarbonyl-amino, (C₁₋₄)alkylcarbonyl, cyano, oxo, hetero(C₃₋₇)cycloalkyl or heteroaryl,
R₃ is hydrogen or (C₁₋₄)alkyl,
X₁ is CH₂,
X₂ is CH₂, O, S, CO, COO, OCO, NHCO, CONH or NR, R being hydrogen or (C₁₋₄)alkyl,
Y is (C₁₋₈)alkylene, (C₁₋₈)alkylenoxy(C₁₋₆)alkylene, (C₁₋₈)alkenylene or (C₁₋₈)alkenylenoxy(C₁₋₆)alkylene,
Ar is a phenyl ring optionally mono-, di- or trisubstituted by, independently, hydroxy or halogen, whereby X₁ and X₂ are in meta or para position to each other,
and either
Z is CO,
AA is a natural or unnatural alpha-amino-acid, and
n is 0 or 1,
or
Z is SO₂,
AA is an optionally substituted ethylenecarbonyl group (derived from a natural or unnatural alpha-amino acid by replacement of N-H in the alpha-amino group by a methylene group), and
n is 1,
in free base form or in acid addition salt form.

3. A compound according to claim 1 of the formula I, wherein
R₁ is (C₁₋₄)alkyl,
R₂ is (C₁₋₆)alkyl,
R₃ is hydrogen or (C₁₋₄)alkyl.
R₄ is hydrogen,
X₁ is CH₂,
X₂ is CH₂ or O,
Y is (C₁₋₈)alkylene,
Ar is unsubstituted phenylene, whereby X₁ and X₂ are in meta position to each other,
and either
Z is CO,
AA is a natural or unnatural alpha-amino-acid, and
n is 0 or 1,
or
Z is SO₂.
AA is an optionally substituted ethylenecarbonyl group (derived from a natural or unnatural alpha-amino acid by replacement of N-H in the alpha-amino group by a methylene group), and
n is 1,
in free base form or in acid addition salt form.

4. A compound according to any one of claims 1 to 3 of the formula 1, wherein
R₁ is (C₁₋₄)alkyl,
R₂ is (C₁₋₈)alkyl,
R₃ is hydrogen or (C₁₋₄)alkyl,
R₄ is hydrogen,
X₁ is CH₂,
X₂ is CH₂ or O,
Y is (C₃₋₆)alkylene,
Ar is unsubstituted phenylene, whereby X₁ and X₂ are in meta position to each other,
and either
Z is CO.
AA is -N(H)-CH(CH₃)-C(O)- or -N(H)-CH₂-C(O)-, and
n is 0 or 1,
or
Z is SO₂,
AA is -CH₂-CH(CH₃)-C(O)- or -CH₂-CH₂C(O)-, and
n is 1,
in free base form or in acid addition salt form.

5. A process for the preparation of a compound as defined in claim 1 of the formula I, or a salt thereof, which includes the steps of
a) for the preparation of a compound of the formula I, wherein Z is CO, cyclisation by amide formation of a compound of the formula 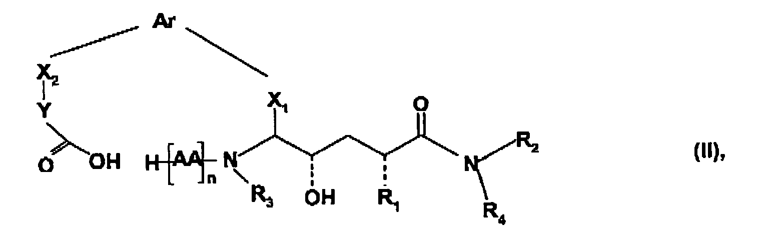 wherein R₁, R₂, R₃, R₄, X₁, X₂, Y, Ar, AA and n are as defined in claim 1, or
b) for the preparation of a compound of the formula I, wherein Z is SO₂ and Y is (C₁₋₈)alkenylene or (C₁₋₈)alkenylenoxy(C₁₋₆)alkylene, cyclisation by metathesis of a compound of the formula 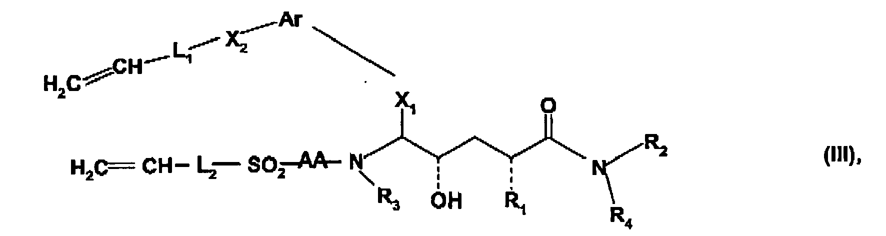 wherein R₁, R₂, R₃, R₄. X₁, X₂, Ar and AA are as defined in claim 1 and L₁ and L₂, independently, are alkylene or alkylenoxyalkylene groups, or
c) for the preparation of a compound of the formula I, wherein Z is SO₂ and Y is (C₁₋₈)alkylene or (C₁₋₆)alkylenoxy(C₁₋₆)arkylene, hydrogenation of a compound of the formula I, wherein Z is SO₂ and Y is (C₁₋₆)alkenylene or (C₁₋₈)alkenylenoxy(C₁₋₆)-alkylene,
and recovering the so obtained compound of the formula I in free base form or in acid addition salt form.

6. A compound according to any one of claims 1 to 4 of the formula I, in free base form or in pharmaceutically acceptable acid addition salt form, for use as a medicament.

7. A compound according to any one of claims 1 to 4 of the formula 1, in free base form or in pharmaceutically acceptable acid addition salt form, for use in the treatment of neurological or vascular disorders related to beta-amyloid generation and/or aggregation.

8. A pharmaceutical composition comprising a compound according to any one of claims 1 to 4 of the formula I, in free base form or in pharmaceutically acceptable acid addition salt form as active ingredient, in association with a pharmaceutical carrier or diluent.

9. The use of a compound according to any one of claims 1 to 4 of the formula I, in free base form or in pharmaceutically acceptable acid addition salt form, for the manufacture of a medicament for the treatment of neurological or vascular disorders related to beta-amyloid generation and/or aggregation.

10. A combination comprising a therapeutically effective amount of a compound according to any one of claims 1 to 4 of the formula I, in free base form or in pharmaceutically acceptable acid addition salt form, and a second drug substance for simultaneous or sequential administration.

## Patentansprüche

1. Verbindung der Formel 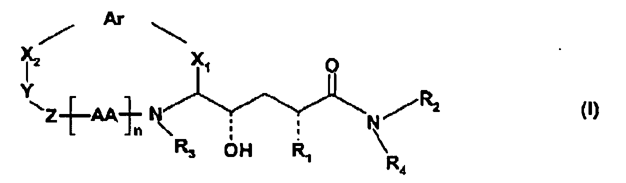 worin
R₁ für (C₁-C₈)-Alkyl, (C₁-C₄)-Alkoxy-(C₁-C₄)-alkyl, Hydroxy-(C₁-C₆)-alkyl, (C₁-C₄)-Alkylthio-(C₁-C₄)-alkyl, (C₁-C₆)-Alkenyl, (C₃-C₇)-Cycloalkyl, (C₃-C₇)-Cycloalkyl-(C₁-C₄)-alkyl, Piperidinyl oder Pyrrolidinyl steht,
R₂ und R₄ unabhängig für Wasserstoff oder optional substituiertes (C₁-C₈)-Alkyl, (C₃-C₇)-Cycloalkyl, (C₃-C₇)-Cycloalkyl-(C₁-C₄)-alkyl, Aryl, Aryl-(C₁-C₄)-alkyl, Heteroaryl oder Heteroaryl-(C₁-C₄)-alkyl stehen, oder
R₂ und R₄ zusammen mit dem Stickstoffatom, an das sie gebunden sind, optional substituiertes Piperidinyl, Pyrrolidinyl, Morpholinyl oder Piperazinyl bilden,
R₃ für Wasserstoff oder (C₁-C₄)-Alkyl steht,
X₁ für CH₂ steht,
X₂ für CH₂, O, S, CO, COO, OCO, NHCO, CONH oder NR steht, wobei R für Wasserstoff oder (C₁-C₄)-Alkyl steht,
Y für (C₁-C₈)-Alkylen, (C₁-C₈)-Alkylenoxy-(C₁-C₆)-alkylen, (C₁-C₈)-Alkenylen oder (C₁-C₈)-Alkenylenoxy-(C₁-C₆)-alkylen steht,
Ar für einen Phenylring steht, der optional monosubstituiert, disubstituiert oder trisubstituiert ist durch unabhängig Hydroxy oder Halogen, wobei sich X₁ und X₂ in meta-Position oder para-Position zueinander befinden, und
entweder
Z für CO steht,
AA für eine natürliche oder nicht natürliche alpha-Aminosäure steht, und
n für 0 oder 1 steht,
oder
Z für SO₂ steht,
AA für optional substituiertes Ethylencarbonyl steht, das von einer natürlichen oder nicht natürlichen Aminosäure unter Ersatz von N-H in der alpha-Aminogruppe durch eine Methylengruppe abgeleitet ist, und
n für 1 steht
in Form der freien Base oder in Form eines Säureadditionssalzes.

2. Verbindung nach Anspruch 1 der Formel I, worin
R₁ für (C₁-C₈)-Alkyl, (C₁-C₄)-Alkoxy-(C₁-C₄)-alkyl, Hydroxy(C₁-C₆)-alkyl, (C₁-C₄)-Alkylthio-(C₁-C₄)-alkyl, (C₁-C₆)-Alkenyl, (C₃-C₇)-Cycloalkyl, (C₃-C₇)-Cycloalkyl-(C₁-C₄)-alkyl, Piperidinyl oder Pyrrolidinyl steht,
R₂ und R₄ unabhängig stehen für
(a) Wasserstoff,
(b) (C₁-C₈)-Alkyl, (C₃-C₇)-Cycloalkyl oder (C₃-C₇)-Cycloalkyl-(C₁-C₄)-alkyl, wobei diese Reste jeweils optional substituiert sind durch ein bis drei Gruppen, die unabhängig ausgewählt sind aus Hydroxy, Hydroxy-(C₁-C₄)-alkyl, (C₁-C₄)-Alkoxy, (C₁-C₄)-Alkoxy-(C₁-C₄)-alkyl, (C₁-C₄)-Alkoxy-(C₁-C₄)-alkoxy, (C₁-C₄)-Alkylsulfanyl, (C₁-C₄)-Alkoxycarbonyl, (C₁-C₄)-Alkylcarbonyloxy, (C₁-C₄)-Alkylcarbonylamino, (C₁-C₄)-Alkylcarbonyl, Cyano, Oxo, Hetero-(C₃-C₇)-cycloalkyl oder Heteroaryl, oder
(c) Aryl, Aryl-(C₁-C₄)-alkyl, Heteroaryl oder Heteroaryl-(C₁-C₄)-alkyl, worin in den letzten beiden Resten das Heteroaryl für einen aromatischen 5- oder 6-gliedrigen Ring steht, worin 1, 2 oder 3 Atome Heteroatome sind, die unabhängig ausgewählt sind aus O, N und S, worin alle Reste optional substituiert sind durch ein bis drei Gruppen, die ausgewählt sind aus Halogen, Hydroxy, Cyano, Trifluormethyl, Carboxy, (C₁-C₄)-Alkyloxycarbonyl, (C₁-C₄)-Alkylcarbamoyl, (C₁-C₄)-Alkylsulfonyl, (C₁-C₄)-Alkylcarbonyloxy, (C₁-C₄)-Alkylcarbonyl, (C₁-C₄)-Alkyl, (C₁-C₄)-Alkoxy oder Hydroxy-(C₁-C₄)-alkyl, oder
R₂ und R₄ zusammen mit dem Stickstoffatom, an das sie gebunden sind für Piperidinyl, Pyrrolidinyl, Morpholinyl oder Piperazinyl stehen, wobei jede dieser Gruppen optional substituiert ist durch ein bis drei Gruppen, die ausgewählt sind aus Hydroxy, Hydroxy-(C₁-C₄)-alkyl, (C₁-C₄)-Alkoxy, (C₁-C₄)-Alkoxy-(C₁-C₄)-alkyl, (C₁-C₄)-Alkoxy-(C₁-C₄)-alkoxy, (C₁-C₄)-Alkylsulfanyl, (C₁-C₄)-Alkoxycarbonyl, (C₁-C₄)-Alkylcarbonyloxy, (C₁-C₄)-Alkylcarbonylamino, (C₁-C₄)-Alkylcarbonyl, Cyano, Oxo, Hetero-(C₃-C₇)-cycloalkyl oder Heteroaryl,
R₃ für Wasserstoff oder (C₁-C₄)-Alkyl steht,
X₁ für CH₂ steht,
X₂ für CH₂, O, S, CO, COO, OCO, NHCO, CONH oder NR steht, wobei R für Wasserstoff oder (C₁-C₄)-Alkyl steht,
Y für (C₁-C₈)-Alkylen, (C₁-C₈)-Alkylenoxy-(C₁-C₆)-alkylen, (C₁-C₈)-Alkenylen oder (C₁-C₈)-Alkenylenoxy-(C₁-C₆)-alkylen steht,
Ar für einen Phenylring steht, der optional monosubstituiert, disubstituiert oder trisubstituiert ist durch unabhängig Hydroxy oder Halogen, wobei sich X₁ und X₂ in meta-Position oder para-Position zueinander befinden, und
entweder
Z für CO steht,
AA für eine natürliche oder nicht natürliche alpha-Aminosäure steht, und
n für 0 oder 1 steht,
oder
Z für SO₂ steht,
AA für optional substituiertes Ethylencarbonyl steht, das von einer natürlichen oder nicht natürlichen Aminosäure unter Ersatz von N-H in der alpha-Aminogruppe durch eine Methylengruppe abgeleitet ist, und
n für 1 steht
in Form der freien Base oder in Form eines Säureadditionssalzes.

3. Verbindung nach Anspruch 1 der Formel I, worin
R₁ für (C₁-C₄)-Alkyl steht,
R₂ für (C₁-C₆)-Alkyl steht,
R₃ für Wasserstoff oder (C₁-C₄)-Alkyl steht,
R₄ für Wasserstoff steht,
X₁ für CH₂ steht,
X₂ für CH₂ oder O steht,
Y für (C₁-C₈)-Alkylen steht,
Ar für unsubstituiertes Phenylen steht, wobei sich X₁ und X₂ in meta-Position zueinander befinden, und
entweder
Z für CO steht,
AA für eine natürliche oder nicht natürliche alpha-Aminosäure steht, und
n für 0 oder 1 steht,
oder
Z für SO₂ steht,
AA für optional substituiertes Ethylencarbonyl steht, das von einer natürlichen oder nicht natürlichen Aminosäure unter Ersatz von N-H in der alpha-Aminogruppe durch eine Methylengruppe abgeleitet ist, und
n für 1 steht
in Form der freien Base oder in Form eines Säureadditionssalzes.

4. Verbindung nach einem der Ansprüche 1 bis 3 der Formel I, worin
R₁ für (C₁-C₄)-Alkyl steht,
R₂ für (C₁-C₆)-Alkyl steht,
R₃ für Wasserstoff oder (C₁-C₄)-Alkyl steht,
R₄ für Wasserstoff steht,
X₁ für CH₂ steht,
X₂ für CH₂ oder O steht,
Y für (C₁-C₈)-Alkylen steht,
Ar für unsubstituiertes Phenylen steht, wobei sich X₁ und X₂ in meta-Position zueinander befinden, und
entweder
Z für CO steht,
AA für -N(H)-CH(CH₃)-C(O)- oder -N(H)-CH₂-C(O)- steht, und
n für 0 oder 1 steht,
oder
Z für SO₂ steht,
AA für -CH₂-CH(CH₃)-C(O)- oder -CH₂-CH₂-C(O)- steht, und
n für 1 steht
in Form der freien Base oder in Form eines Säureadditionssalzes.

5. Verfahren zur Herstellung einer Verbindung gemäß der Definition im Anspruch 1 der Formel I oder eines Salzes hiervon durch die Stufen
(a) zur Herstellung einer Verbindung der Formel I, worin Z für CO steht, Cyclisierung der Amidbildung einer Verbindung der Formel 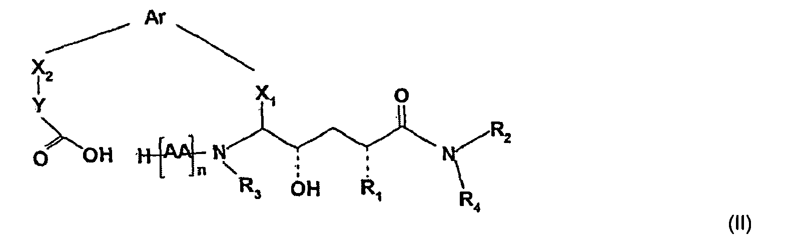 worin R₁. R₂, R₃, R₄, X₁, X₂, Y, Ar, AA und n wie im Anspruch 1 definiert sind, oder
(b) zur Herstellung der Formel I, worin Z für SO₂ steht und Y für (C₁-C₈)-Alkenylen oder (C₁-C₈)-Alkenylenoxy-(C₁-C₆)-alkylen steht, Cyclisierung durch Metathese einer Verbindung der Formel 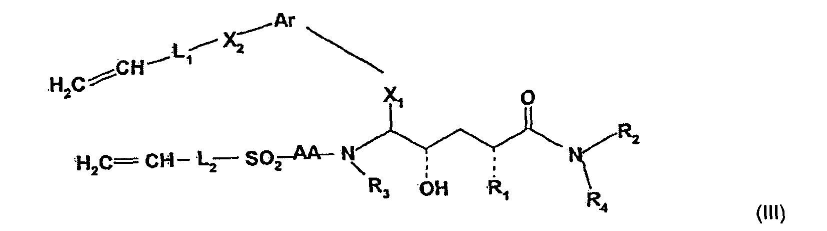 worin R₁, R₂, R₃, R₄, X₁, X₂, Ar und AA wie im Anspruch 1 definiert sind und L₁ und L₂ unabhängig für Alkylen oder Alkylenoxyalkylen stehen, oder
(c) zur Herstellung einer Verbindung der Formel I, worin Z für SO₂ steht und Y für (C₁-C₈)-Alkylen oder (C₁-C₈)-Alkylenoxy-(C₁-C₆)-alkylen steht, Hydrierung einer Verbindung der Formel I, worin Z für SO₂ steht und Y für (C₁-C₈)-Aikenylen oder (C₁-C₈)-Alkenyfenoxy-(C₁-C₆)-alkylen steht,
und Gewinnung der so erhaltenen Verbindung der Formel I in Form der freien Base oder in Form eines Säureadditionssalzes.

6. Verbindung nach einem der Ansprüche 1 bis 4 der Formel I in Form der freien Base oder in Form eines pharmazeutisch akzeptablen Säureadditionssalzes zur Verwendung als ein Arzneimittel.

7. Verbindung nach einem der Ansprüche 1 bis 4 der Formel I in Form der freien Base oder in Form eines pharmazeutisch akzeptablen Säureadditionssalzes zur Verwendung für die Behandlung neurologischer oder vaskularer Störungen, die mit einer Bildung von beta-Amyloid und/oder einer Aggregation hiervon zusammenhängen.

8. Pharmazeutische Zusammensetzung, umfassend eine Verbindung nach einem der Ansprüche 1 bis 4 der Formel I in Form der freien Base oder in Form eines pharmazeutisch akzeptablen Säureadditionssalzes als Wirkstoff, in Assoziation mit einem pharmazeutischen Träger oder Verdünnungsmittel.

9. Verwendung einer Verbindung nach einem der Ansprüche 1 bis 4 der Formel I in Form der freien Base oder eines pharmazeutisch akzeptablen Säureadditionssalzes hiervon zur Herstellung eines Arzneimittels für die Behandlung neurologischer oder vaskularer Störungen, die mit einer Bildung von beta-Amyloid und/oder einer Aggregation hiervon zusammenhängen.

10. Kombination, umfassend eine therapeutisch wirksame Menge einer Verbindung nach einem der Ansprüche 1 bis 4 der Formel I in Form der freien Base oder in Form eines pharmazeutisch akzeptablen Säureadditionssalzes und einen zweiten Wirkstoff, zur simultanen oder sequentiellen Verabreichung.

## Revendications

1. Composé de formule 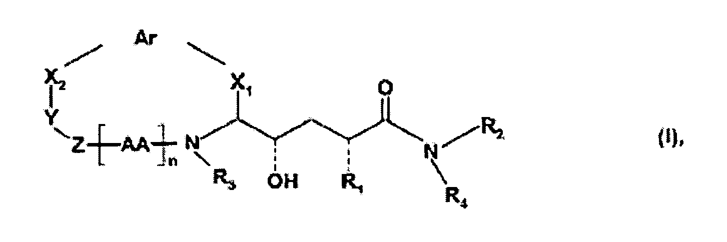 dans lequel
R₁ est un alkyle en (C₁₋₈), un alcoxy en (C₁₋₄)-alkyle en (C₁₋₄), un hydroxy-alkyle en (C₁₋₆), un alkylthio en (C₁₋₄)-alkyle en (C₁₋₄), un alcényle en (C₁₋₆), un cycloalkyle en (C₃₋₇), un cycloalkyle en (C₃₋₇)-alkyle en (C₁₋₄), un pipéridinyle ou un pyrrolidinyle,
R₂ et R₄, indépendamment, sont un hydrogène ou un groupe éventuellement substitué de type alkyle en (C₁₋₆), cycloalkyle en (C₃₋₇), cycloalkyle en (C₃₋₇)-alkyle en (C₁₋₄), aryle, aryle-alkyle en (C₁₋₄), hétéroaryle ou hétéroaryle-alkyle en (C₁₋₄), ou
R₂ et R₄, conjointement avec l'atome d'azote auquel ils sont liés, forment un groupe éventuellement substitué de type pipéridinyle, pyrrolidinyle, morpholinyle ou pipérazinyle,
R₃ est un hydrogène ou un alkyle en (C₁₋₄),
X₁ est CH₂,
X₂ est CH₂, O, S, CO, COO, OCO, NHCO, CONH ou NR, R étant un hydrogène ou un alkyle en (C₁₋₄),
Y est un alkylène en (C₁₋₈), un alkylènoxy en (C₁₋₈)-alkylène en (C₁₋₆), un alcénylène en (C₁₋₈) ou un alcénylènoxy en (C₁₋₈)-alkylène en (C₁₋₆),
Ar est un cycle phényle éventuellement mono-, di- ou tri-substitué par, indépendamment, un hydroxy ou un halogène, au moyen duquel X₁ et X₂ sont en position méta ou para l'un par rapport à l'autre,
et soit
Z est CO,
AA est un alpha-acide aminé naturel ou non naturel, et
n est égal à 0 ou à 1,
soit
Z est SO₂,
AA est un groupe éthylènecarbonyle éventuellement substitué (dérivé d'un alpha-acide aminé naturel ou non naturel par remplacement de N-H dans le groupe alpha-amino par un groupe méthylène), et
n est égal à 1,
sous forme de base libre ou sous forme de sel d'addition acide.

2. Composé de formule 1 selon la revendication 1, dans lequel
R₁ est un alkyle en (C₁₋₈), un alcoxy en (C₁₋₄)-alkyle en (C₁₋₄), un hydroxy-alkyle en (C₁₋₆), un alkylthio en (C₁₋₄)-alkyle en (C₁₋₄), un alcényle en (C₁₋₆), un cycloalkyle en (C₃₋₇), un cycloalkyle en (C₃₋₇)-alkyle en (C₁₋₄), un pipéridinyle ou un pyrrolidinyle,
R₂ et R₄, indépendamment, sont
(a) un hydrogène
(b) un alkyle en (C₁₋₈), un cycloalkyle en (C₃₋₇) ou un cycloalkyle en (C₃₋₇)-alkyle en (C₁₋₄), dans chaque cas éventuellement substitué par de un à trois groupes choisis parmi hydroxy, hydroxy-alkyle en (C₁₋₄), alcoxy en (C₁₋₄), alcoxy en (C₁₋₄)-alkyle en (C₁₋₄), alcoxy en (C₁₋₄)-alcoxy en (C₁₋₄), alkylsulfanyle en (C₁₋₄), alcoxycarbonyle en (C₁₋₄), alkylcarbonyloxy en (C₁₋₄), alkylcarbonylamino en (C₁₋₄), alkylcarbonyle en (C₁₋₄), cyano, oxo, hétéro-cycloalkyle en (C₃₋₇) ou hétéroaryle ou
(c) un aryle, un aryle-alkyle en (C₁₋₄), un hétéroaryle ou un hétéroaryle-alkyle en (C₁₋₄), dans les deux derniers radicaux, hétéroaryle désignant un cycle aromatique à 5 ou 6 chaînons, dans lequel 1, 2 ou 3 atomes sont des hétéroatomes indépendamment choisis parmi O, N et S, tous les radicaux étant éventuellement substitués par de un à trois groupes choisis parmi halogène, hydroxy, cyano, trifluorométhyle, carboxy, alkyloxycarbonyle en (C₁₋₄), alkyle en (C₁₋₄)-carbamoyle, alkylsulfonyle en (C₁₋₄), alkylcarbonyloxy en (C₁₋₄), alkylcarbonyle en (C₁₋₄), alkyle en (C₁₋₄), alcoxy en (C₁₋₄) ou hydroxy-alkyle en (C₁₋₄), ou
R₂ et R₄, conjointement avec l'atome d'azote auquel ils sont liés, forment un groupe pipéridinyle, pyrrolidinyle, morpholinyle ou pipérazinyle, chacun de ceux-ci étant éventuellement substitué par de un à trois groupes choisis parmi hydroxy, hydroxy-alkyle en (C₁₋₄), alcoxy en (C₁₋₄), alcoxy en (C₁₋₄)-alkyle en (C₁₋₄), alcoxy en (C₁₋₄)-alcoxy en (C₁₋₄), alkylsulfanyle en (C₁₋₄), alcoxycarbonyle en (C₁₋₄), alkylcarbonyloxy en (C₁₋₄), alkylcarbonylamino en (C₁₋₄), alkylcarbonyle en (C₁₋₄), cyano, oxo, hétéro-cycloalkyle en (C₃₋₇) ou hétéroaryle,
R₃ est un hydrogène ou un alkyle en (C₁₋₄),
X₁ est CH₂,
X₂ est CH₂, O, S, CO, COO, OCO, NHCO, CONH ou NR, R étant un hydrogène ou un alkyle en (C₁₋₄),
Y est un alkylène en (C₁₋₈), un alkylènoxy en (C₁₋₈)-alkylène en (C₁₋₆), un alcénylène en (C₁₋₈) ou un alcénylènoxy en (C₁₋₈)-alkylène en (C₁₋₆),
Ar est un cycle phényle éventuellement mono-, di- ou tri-substitué par, indépendamment, un hydroxy ou un halogène, au moyen duquel X₁ et X₂ sont en position méta ou para l'un par rapport à l'autre,
et soit
Z est CO,
AA est un alpha-acide aminé naturel ou non naturel, et
n est égal à 0 ou à 1,
soit
Z est SO₂,
AA est un groupe éthylènecarbonyle éventuellement substitué (dérivé d'un alpha-acide aminé naturel ou non naturel par remplacement de N-H dans le groupe alpha-amino par un groupe méthylène), et
n est égal à 1,
sous forme de base libre ou sous forme de sel d'addition acide.

3. Composé de formule I selon la revendication 1, dans lequel
R₁ est un alkyle en (C₁₋₄),
R₂ est un alkyle en (C₁₋₆),
R₃ est un hydrogène ou un alkyle en (C₁₋₄),
R₄ est un hydrogène,
X₁ est CH₂,
X₂ est CH₂ ou O,
Y est un alkylène en (C₁₋₈),
Ar est un phénylène non substitué, au moyen duquel X₁ et X₂ sont en position méta l'un par rapport à l'autre,
et soit
Z est CO,
AA est un alpha-acide aminé naturel ou non naturel, et
n est égal à 0 ou à 1,
soit
Z est SO₂,
AA est un groupe éthylènecarbonyle éventuellement substitué (dérivé d'un alpha-acide aminé naturel ou non naturel par remplacement de N-H dans le groupe alpha-amino par un groupe méthylène), et
n est égal à 1,
sous forme de base libre ou sous forme de sel d'addition acide.

4. Composé de formule I selon l'une quelconque des revendications 1 à 3, dans lequel
R₁ est un alkyle en (C₁₋₄),
R₂ est un alkyle en (C₁₋₆),
R₃ est un hydrogène ou un alkyle en (C₁₋₄),
R₄ est un hydrogène,
X₁ est CH₂,
X₂ est CH₂ ou O,
Y est un alkylène en (C₃₋₆),
Ar est un phénylène non substitué, au moyen duquel X₁ et X₂ sont en position méta l'un par rapport à l'autre,
et soit
Z est CO,
AA est -N(H)-CH(CH₃)-C(O)- ou -N(H)-CH₂-C(O)-, et
n est égal à 0 ou à 1,
soit
Z est SO₂,
AA est -CH₂-CH(CH₃)-C(O)- ou -CH₂-CH₂-C(O)-, et
n est égal à 1,
sous forme de base libre ou sous forme de sel d'addition acide.

5. Processus de préparation d'un composé de formule I tel que défini dans la revendication 1, ou d'un sel de celui-ci, qui comprend les étapes de
a) pour la préparation d'un composé de formule I dans lequel Z est CO, cyclisation, par formation d'amide, d'un composé de formule 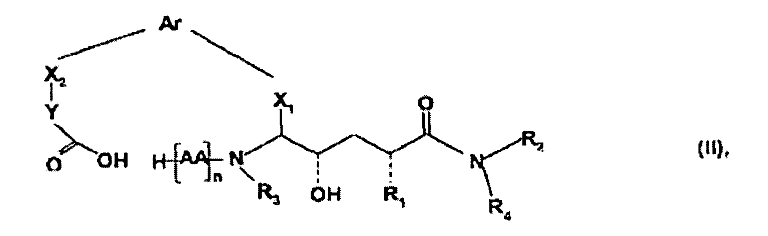 dans lequel R₁, R₂, R₃, R₄, X₁, X₂, Y, Ar, AA et n sont tels que définis dans la revendication 1, ou
b) pour la préparation d'un composé de formule 1 dans lequel Z est SO₂ et Y est un alcénylène en (C₁₋₈) ou un alcénylènoxy en (C₁₋₈)-alkylène en (C₁₋₆), cyclisation, par métathèse, d'un composé de formule 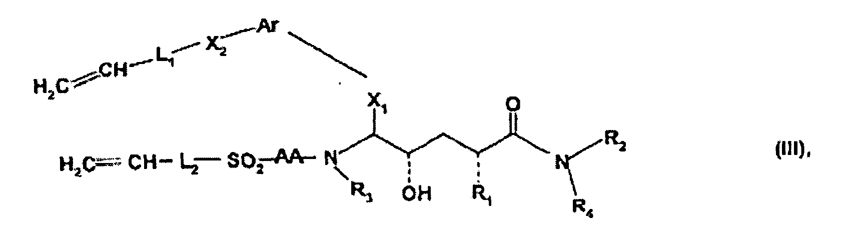 dans lequel R₁, R₂, R₃, R₄, X₁, X₂, Ar et AA sont tels que définis dans la revendication 1 et L₁ et L₂, indépendamment, sont des groupes alkylène ou alkylènoxyalkylène, ou
c) pour la préparation d'un composé de formule I dans lequel Z est SO₂ et Y est un alkylène en (C₁₋₈) ou un alkylènoxy en (C₁₋₈)-alkylène en (C₁₋₆), hydrogénation d'un composé de formule I dans lequel Z est SO₂ et Y est un alcénylène en (C₁₋₈) ou un alcénylènoxy en (C₁₋₈)-alkylène en (C₁₋₆),
et récupération du composé de formule I, ainsi obtenu, sous forme de base libre ou sous forme de sel d'addition acide.

6. Composé de formule I selon l'une quelconque des revendications 1 à 4, sous la forme d'une base libre ou sous la forme d"un sel d'addition acide pharmaceutiquement acceptable, pour utilisation en tant que médicament.

7. Composé de formule I selon l'une quelconque des revendications 1 à 4, sous la forme d'une base libre ou sous la forme d'un sel d'addition acide pharmaceutiquement acceptable, pour utilisation dans le traitement de troubles neurologiques ou vasculaires en rapport avec la formation et/ou l'agrégation de bêta-amyloïdes.

8. Composition pharmaceutique comprenant un composé de formule I selon l'une quelconque des revendications 1 à 4, sous la forme d'une base libre ou sous la forme d'un sel d'addition acide pharmaceutiquement acceptable en tant que principe actif, en association avec un véhicule ou avec un diluant pharmaceutique.

9. Utilisation d'un composé de formule I selon l'une quelconque des revendications 1 à 4, sous forme de base libre ou sous forme de sel d'addition acide pharmaceutiquement acceptable, pour la fabrication d'un médicament pour le traitement de troubles neurologiques ou vasculaires en rapport avec la formation et/ou l'agrégation de bêta-amyloïdes.

10. Combinaison comprenant une quantité thérapeutiquement efficace d'un composé de formule I selon l'une quelconque des revendications 1 à 4, sous forme de base libre ou sous forme de sel d'addition acide pharmaceutiquement acceptable, et une seconde substance médicamenteuse pour administration simultanée ou séquentielle.
